(19) Europäisches Patentamt
European Patent Office
Office européen des brevets

(11) Numéro de publication: **0 357 510 A1**

# (12) DEMANDE DE BREVET EUROPEEN

(21) Numéro de dépôt: **89402377.9**

(22) Date de dépôt: **31.08.89**

(51) Int. Cl.⁵: **C 07 K 5/02**
C 07 K 7/02, C 07 K 5/10,
C 07 K 7/06, A 61 K 37/64

(30) Priorité: **02.09.88 FR 8811528**

(43) Date de publication de la demande:
**07.03.90 Bulletin 90/10**

(84) Etats contractants désignés:
**AT BE CH DE ES FR GB GR IT LI LU NL SE**

(71) Demandeur: **CENTRE NATIONAL DE LA RECHERCHE SCIENTIFIQUE (CNRS)**
**15, Quai Anatole France**
**F-75700 Paris Cedex 07 (FR)**

(72) Inventeur: **Wakselmann, Michel**
**47, rue Barrault**
**F-75013 Paris (FR)**

**Mazaleyrat, Jean-Paul**
**28, rue Camille Desmoulins**
**F-94600 Choisy-le-Roi (FR)**

**Reboud, Michèle**
**16, rue des Orchidées**
**F-75013 Paris (FR)**

(74) Mandataire: **Mongrédien, André et al**
**c/o BREVATOME 25, rue de Ponthieu**
**F-75008 Paris (FR)**

## (54) Dérivés de cyclopeptides utilisables comme inhibiteurs sélectifs, vis-à-vis de protéases à sérine active.

(57) L'invention concerne des dérivés de cyclopeptides utilisables comme inhibiteurs de protéases qui répondent à la formule :

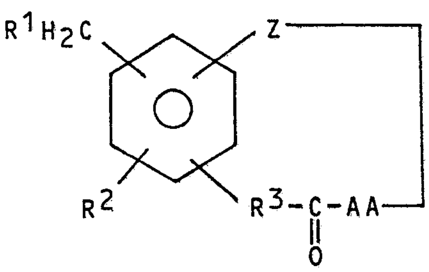

(I)

ans laquelle $R^1$ est un atome d'halogène, par exemple le brome ou un radical tel que $S^+R_2^4X_{1/v}^{v-}$ $R^2$ est un atome d'hydrogène, un radical alkyle, un atome d'halogène ou d'autres groupements, $R^3$ est NH, O ou S, Z comprend une séquence peptidique $Z^1$ et un groupement tel que CO à son extrémité liée au noyau aromatique, et CO-AA est un radical dérivé d'un aminoacide ayant une spécificité vis-à-vis de la protéase à inhiber.

Ces dérivés de cyclopeptides peuvent être utilisés dans des compositions pharmaceutiques.

EP 0 357 510 A1

**Description**

**DERIVES DE CYCLOPEPTIDES, UTILISABLES COMME INHIBITEURS SELECTIFS, VIS-A-VIS DE PROTEASES A SERINE ACTIVE**

La présente invention a pour objet de nouveaux dérivés de cyclopeptides, utilisables comme inhibiteurs de protéases.

Elle s'applique en particulier aux protéases à sérine ou à cystéine active telles que les facteurs IXa, Xa, XIa, XIIa et VIIa de coagulation du sang, la thrombine, la kallikréine du plasma, la protéine C activée, les facteurs C1r, C1s, D et B d'activation en complément, la $C_3$ convertase, la trypsine, la chymotrypsine, l'élastase, l'entérokinase, la plasmine, les activateurs du plasminogène (urokinase, tpA), l'acrosine, la cathepsine G, les chymases, les tryptases et les protéases dépendantes de l'ATP.

De façon plus précise, elle concerne de nouveaux dérivés de cyclopeptides susceptibles d'agir sélectivement comme inhibiteurs vis-à-vis d'une protéase donnée.

On sait que les protéases à sérine ou à cystéine active sont impliquées dans de très nombreux processus physiologiques. Des états pathologiques peuvent s'instaurer lorsqu'il y a déséquilibre entre une protéase et ses inhibiteurs macromoléculaires naturels. Pour pallier ce déséquilibre, des inhibiteurs synthétiques pourraient être utilisés et auraient de ce fait un grand intérêt en thérapeutique, par exemple dans les pathologies suivantes :

- emphysème pulmonaire, arthrite rhumatoïde, inflammation et vieillissement cutané où la protéase active est l'élastase leucocytaire, la cathepsine G intervient également ;
- invasion tumorale et processus métastasique lié à la présence de protéases telles que les activateurs du plasminogène ou la plasmine ;
- action antithrombotique et prévention des infarctus cérébraux et coronaires où la protéase responsable est la thrombine ;
- contrôle de la thrombolyse et de la fertilité qui concernent les activateurs du plasminogène et la plasmine ; et
- lutte antivirale et lutte antiparasitaire car certains virus produisent des protéases à cystéine active.

Aussi, depuis quelques années, de nombreuses recherches ont été effectuées pour synthétiser des inhibiteurs irréversibles des protéases. Parmi ceux-ci, on connaît en particulier des inhibiteurs synthétiques qui sont des dérivés de coumarines comme la 3,4-dihydro- 3,4-dibromo-6 bromométhylcoumarine qui est un inactivateur irréversible très efficace vis-à-vis des protéases à sérine et à cystéine, mais qui est malheureusement dépourvue de sélectivité. Ces dérivés de coumarine sont en particulier décrits dans le livre "proteinase inhibitors" de A. J. Barrett et G. Salvesen, Elsevier, 1986, p. 119-121.

Aussi, il serait d'un grand intérêt de synthétiser d'autres inhibiteurs des protéases qui soient spécifiques d'une protéase donnée et permettent d'inactiver celle-ci sans agir sur les protéases analogues.

La présente invention a précisément pour objet un nouveau dérivé de cyclopeptide qui permet d'atteindre ce résultat.

Selon l'invention, le dérivé de cyclopeptide répond à la formule :

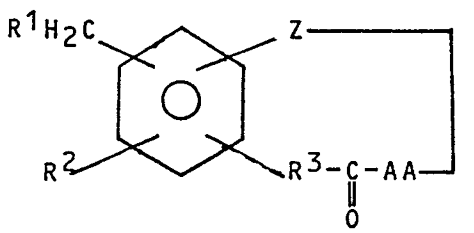

(I)

dans laquelle :

- $R^1$ est choisi parmi les atomes de fluor, de chlore, de brome et d'iode, et les radicaux $OSO_2R^4$, $OP(O)R^4{}_2$, $OC(O)R^4$ et $S^+R^4{}_2X^{v-}_{1/v}$ avec $R^4$ représentant un radical alkyle, perfluoroalkyle ou aryle, les $R^4$ pouvant être différents , et $X^-$ représentant un anion de valence v;
- $R^2$ est un atome d'hydrogène, un radical alkyle, un atome d'halogène, $NO_2$, $COOR^5$, $CF_3$, CN ou $SO_2R^5$ avec $R^5$ représentant un radical alkyle ou un radical aryle ;
- $R^3$ représente un atome d'oxygène, un atome de soufre ou -NH- ;
- Z comprend une séquence peptidique $Z_1$ d'aminoacides ou d'analogues identiques ou différents et un groupement choisi parmi $-(CH_2)_n$, $-0(CH_2)_n-$ ou CO- à son extrémité liée au noyau aromatique avec n étant un nombre entier de 1 à 8 ;

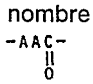

est un radical dérivé d'un aminoacide ou d'un analogue d'aminoacide de formule :

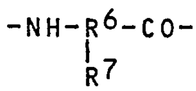

dans laquelle :
$R^6$ représente -N- ou -CH-, et
$R^7$ représente un radical choisi parmi H, $-CH_3$ , $-CH_2CH(CH_3)_2$ ;

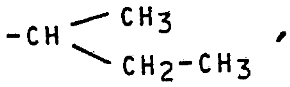

$-CH_2-CO-NH_2$, $-CH_2-CH_2-COOH$, $-CH_2-CH_2-CO-NH_2-$, $(CH_2)_4-NH_2$, $-CH_2OH$, $-CH_2-SH$

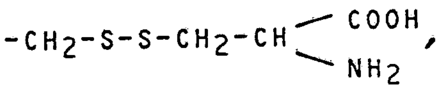

$-CH_2-C_6H_5$, $-CH_2-C_6H_4OH$, $-(CH_2)_3NHC(=NH)-NH_2$, $-CH_2-COOH$, $-CHOH-CH_3$

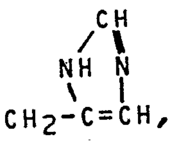

$(CH_2)SCH_3$,

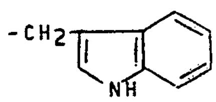

et $-CH(CH_3)_2$
ou de formule :

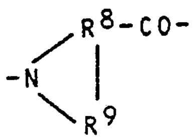

dans laquelle $R^8$ représente N ou CH et $R^9$ représente le radical $-(CH_2)_3$-ou $-CH_2-CHOH-CH_2$.

L'invention porte également sur les sels d'addition à des acides pharmaceutiquement acceptables de ces dérivés de cyclopeptides.

A titre d'exemple, on peut utiliser de tels sels lorsque $R^7$ comprend un groupement amino $NH_2$.

Les acides pharmaceutiquement acceptables peuvent être par exemple HCl, HBr, $PO_4H_3$, $R^4COOH$, $SO_4H_2$, $R^4SO_3H$ avec $R^4$ ayant la signification donnée ci-dessus.

Dans cette formule les radicaux alkyle utilisés ont généralement de 1 à 5 atomes de carbone et peuvent être linéaires ou ramifiés. Les radicaux aryle peuvent avoir de 6 à 14 atomes de carbone. A titre d'exemples de tels radicaux, on peut citer les radicaux méthyle, éthyle, phényle, naphtyle.

Dans le dérivé de cyclopeptide de l'invention, le groupe $CH_2R^1$ permet d'inactiver la protéase et le choix de la séquence peptidique $Z_1$ ainsi que de l'acide aminé C(O)-AA permet de conférer à l'ensemble la sélectivité voulue vis-à-vis d'une protéase donnée.

De préférence, la séquence peptidique $Z_1$ comprend de 2 à 8 aminoacides ou analogues d'aminoacides, identiques ou différents.

Les aminoacides et les analogues d'aminoacides susceptibles d'être utilisés sont par exemple ceux qui répondent à la formule

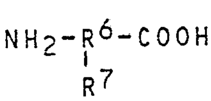

dans laquelle $R^6$ et $R^7$ ont la signification donnée ci-dessus.

De préférence, dans l'invention, $R^3$ est en position ortho ou méta par rapport à Z et en position ortho ou para par rapport à $R^1H_2C$. Cette disposition permet en effet d'obtenir les meilleurs résultats en ce qui concerne la capacité du groupe $CH_2R_1$ d'inactiver la protéase, et la capacité de la chaîne cyclopeptidique de s'adapter sélectivement à la protéase à inactiver.

Le mécanisme d'inactivation correspond tout d'abord à une coupure du cyclopeptide entre $R^3$ et l'acide aminé C(O)-AA, ce qui permet une fixation du cyclopeptide sur la sérine active de la protéase par le groupe C(O)-AA en formant ainsi un acylenzyme. Cette coupure du cyclopeptide confère à $R^1$ une mobilité accrue par formation d'un dérivé intermédiaire du type méthylène quinonimine lorsque $R^3$ représente NH, qui se

transforme ensuite en méthylène aniline avec fixation sur un résidu d'aminoacide du site actif de l'enzyme par le groupement méthylène, ce qui assure ainsi une inactivation de l'enzyme.

De préférence, $R^1$ est Cl, Br, $OSO_2R^4$ ou mieux encore $S^+R_4{}^2X^{v-}_{1/v}$. $X^-$ peut être un anion dérivé d'un acide pharmaceutiquement acceptable tel que $Cl^-$, $ClO_4^-$, $Br^-$, $BF_4^-$,$PF_6^-$ , $R^4COO^-$, $R^4SO_3$ avec $R^4$ ayant la signification donnée ci-dessus, par exemple $CF_3COO^-$. Dans $S^+R^2{}_4X^{v-}_{1/v}$ , les deux $R^4$ peuvent être identiques ou différents et sont de préférence des radicaux alkyle ou aryle.

Dans le cyclopeptide de l'invention, Z et AAC(O) sont choisis en fonction de la protéase à inactiver.

-AA-C(O)- est dérivé d'un acide aminé qui correspond à la spécificité primaire de la classe de protéases visée .

Dans le cas où l'on veut inactiver une protéase du type chymotrypsine, -AA-C(O)- est de préférence dérivé de la phénylalanine, de la tyrosine, du tryptophane ou de la méthionine, et correspond à la formule :

$-NH-R^6-CO-$
  |
  $R^7$

dans laquelle $R^6$ est CH, et $R^7$ est $CH_2$-$C_6H_5$, $CH_2$-$C_6H_4OH$,

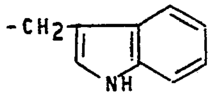

ou $(CH_2)_2SCH_3$ :

Dans le cas où la protéase à inhiber appartient au genre trypsine, -AA-C(O)- est de préférence dérivé de la lysine ou de l'arginine et répond à la formule :

$-NH-R^6-CO-$
  |
  $R^7$

dans laquelle $R^6$ est CH, et $R^7$ est $-(CH_2)_4NH_2$ ou $-CH_2)_3NH-C(=NH)-NH_2$.

Lorsque la protéase à inactiver appartient au genre élastase, -AA-C(O)- est de préférence dérivé de l'alanine ou de la valine et répond à la formule :

$-NH-R^6-CO-$
  |
  $R^7$

dans laquelle $R^6$ est CH et $R^7$ est $-CH_3$ ou $-CH(CH_3)_2$.

Selon l'invention, la chaîne covalente Z est choisie également en fonction de la protéase à inactiver. Cette chaîne tient compte de l'affinité présentée par les sous-sites de fixation des substrats naturels de la protéase à inactiver. Les protéases ont en effet des centres actifs étendus et l'on peut augmenter l'efficacité et la spécificité de l'inhibiteur en choisissant une chaîne covalente Z remplissant les conditions de spécificité des sous-sites de fixation.

Dans le cas où la protéase est du genre trypsine, chymotrypsine ou élastase, on peut remplir cette condition de spécificité avec Z répondant à la formule :

      $R^7$
      |
$-C(O)-(NH-R^6-CO)_m-$

dans laquelle m = 4, 5 ou 6 et $R^6$ et $R^7$ ont la signification donnée ci-dessus.

Dans le cyclopeptide de l'invention, le noyau benzénique qui fait partie du cyclopeptide peut comporter de plus un substituant $R^2$ qui ne modifie pas le mécanisme d'inactivation de la protéase par le groupement $CH_2$. Ce substituant $R^2$ peut être un atome d'hydrogène, un atome d'halogène ou différents radicaux comme on l'a vu précédemment.

La présente invention a également pour objet des compositions pharmaceutiques ayant la propriété d'inhiber une protéase donnée, qui comprennent une quantité pharmaceutiquement acceptable d'un dérivé de cyclopeptide conforme à l'invention.

Ces compositions pharmaceutiques peuvent être sous la forme de solutions, de suspensions, de poudres ou de granules solubilisables, de sirops ou d'élixirs, de gouttes auriculaires, nasales ou ophtalmiques, de comprimés, de gélules, d'aérosols, de pommades, d'applications transdermiques ou de suppositoires, dans des présentations dosées contenant des supports non toxiques, des adjuvants et des excipients. Les injections peuvent être par exemple intraveineuses, intramusculaires, sous-cutanées, intradermiques, intrasternales, intra-articulaires ; des techniques d'infusion ou des techniques d'instillation (intratrachéale par

exemple) peuvent être utilisées.

Les préparations pour usage oral peuvent contenir un ou plusieurs agents de sucrage, agents d'aromatisation et agents de conservation. Les comprimés contiennent la molécule active de dérivé de cyclopeptide mélangée à des excipients non toxiques et acceptables sur le plan pharmaceutique. Parmi les excipients, on peut citer par exemple des diluants inertes comme le carbonate de calcium ou de sodium, le phosphate de calcium ou de sodium, le lactose ; des agents permettant la granulation et l'effritement, par exemple, l'amidon de maïs ; des agents de fixation, par exemple la gélatine, l'amidon ; des agents de lubrification, par exemple le talc ou le stéarate de magnésium. Les comprimés peuvent être enrobés ou non enrobés (par exemple à l'aide de monostéarate ou de distéarate de glycérol) afin de retarder leur désintégration et leur absorption.

Les gélules peuvent présenter une capsule de gélatine dure contenant la molécule active mélangée à un solide inerte (kaolin, carbonate de calcium par exemple), ou une capsule de gélatine souple dans laquelle le dérivé de cyclopeptide est mélangé à de l'eau ou à un corps gras (paraffine liquide par exemple).

Des suspensions aqueuses contenant des dérivés de cyclopeptides et des excipients convenables, avec éventuellement un ou plusieurs agents de conservation (par exemple, l'éthyl p-hydroxybenzoate), agents de coloration, agents sucrés et agents d'aromatisation peuvent être réalisées. Parmi les excipients, on peut citer des agents de suspensions (par exemple, la méthylcellulose, la gomme d'acacia), des agents de dispersion ou de mouillage tels que, par exemple, des phosphatides naturels (exemple : lécithine) ou des produits de condensation de l'oxyde d'éthylène avec divers esters partiels d'acides gras ou alcools aliphatiques. Des suspensions huileuses de la molécule active peuvent être préparées en utilisant une huile végétale (huile d'olive par exemple) ou une huile minérale (paraffine liquide par exemple), en présence éventuellement d'agents de sucrage, d'aromatisation comme ceux donnés en exemple plus haut et d'agents de conservation (en particulier, un antioxydant).

Sirops et élixirs pourront contenir des agents de sucrage (sorbitol, sucrose par exemple), un ou plusieurs agents de conservation et d'aromatisation. Des granules ou des poudres susceptibles d'être mises en suspension dans l'eau peuvent être obtenues par mélange des dérivés de cyclopeptides avec un agent mouillant ou dispersif, un ou plusieurs agents de conservation et divers excipients. Des émulsions des dérivés de cyclopeptides dans l'eau peuvent être réalisées en utilisant une huile minérale ou végétale et divers agents émulsifiants tels que, par exemple, des gommes naturelles, des phosphatides naturels, divers acides gras estérifiés.

Les dérivés de cyclopeptides peuvent aussi être présentés sous forme de suspensions stériles injectables, aqueuses ou huileuses, utilisant les agents de suspension ou de mouillage décrits plus haut. Les solvants ou diluants ou excipients peuvent être par exemple le 1,3-butanediol, une solution isotonique de chlorure de sodium, l'eau, etc. ... Les suppositoires contenant le principe actif peuvent être préparés avec les excipients habituels dans ce domaine tels que le polyéthylène glycol ou le beurre de cacao par exemple. Pour les usages locaux, des pommades, des crèmes, des gelées, des suspensions, des solutions, etc. ... contenant le principe actif peuvent être préparées.

Des doses de 0,1 à 40mg par kilogramme de poids corporel et par jour peuvent être proposées étant entendu cependant que la dose pour un patient donné peut dépendre d'un certain nombre de facteurs tels que par exemple l'efficacité du dérivé de cyclopeptides concerné, l'âge, le poids, le mode d'administration, le régime, les interactions médicamenteuses, la gravité de la maladie.

Ces compositions sont utilisables dans les pathologies évoquées précédemment.

Les dérivés de cyclopeptides de l'invention peuvent être préparés par des procédés classiques.

Ainsi, on peut préparer les dérivés de cyclopeptides dans lesquels $R^3$ représente NH à partir d'un dérivé de formule (II) :

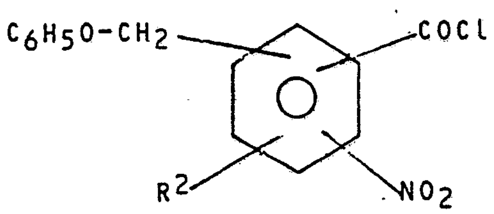

(II)

en réalisant les étapes successives suivantes.

a) Réaction du composé de formule (II) avec $HZ^{10}C_2H_5$ pour obtenir le composé de formule (III) :

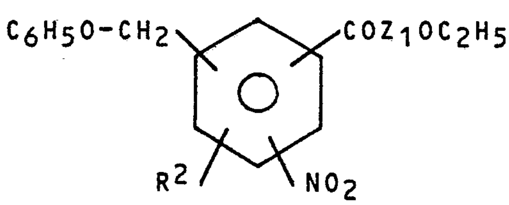

(III)

b) Réaction du composé de formule (III) avec l'hydrogène pour obtenir le composé de formule (IV) :

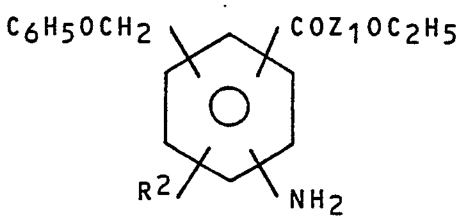

(IV)

c) Réaction du composé de formule (IV) avec l'aminoacide protégé correspondant à AA-C(O) qui répond par exemple à la formule :

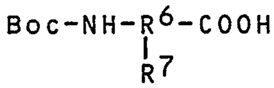

dans laquelle Boc représente le groupement protecteur tertiobutyloxycarbonyle.
Cette réaction peut être effectuée en utilisant la dicyclohexylcarbodiimide (DCC) et elle conduit au composé suivant de formule (V) :

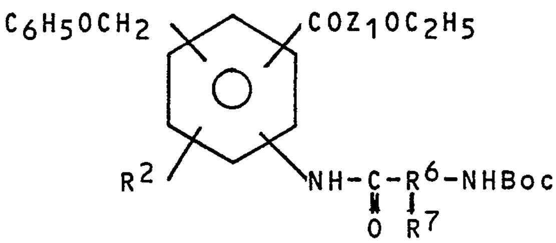

(V)

d) Réaction du composé de formule V avec l'hydrate d'hydrazine dans un solvant tel que le méthanol, pour obtenir le composé de formule (VI) :

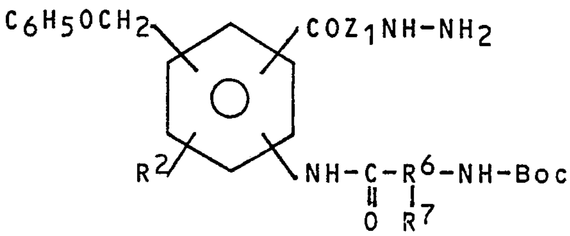

(VI)

e) Réaction du composé de formule (VI) avec l'acide trifluoroacétique pour obtenir le composé de formule (VII) :

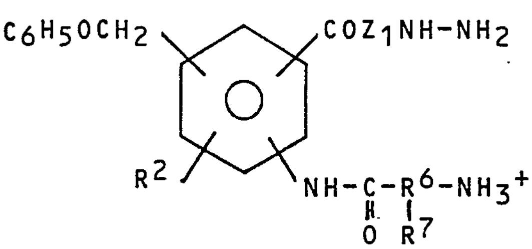

(VII)

f) Réaction du composé de formule (VII) avec l'acide nitreux ou un nitrite d'alkyle pour obtenir le composé de formule (VIII) :

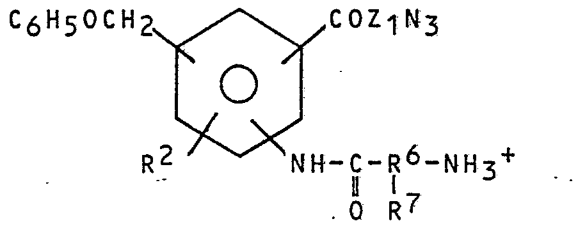

(VIII)

g) Réaction du composé de formule (VIII) avec une amine tertiaire, telle que la diisopropyléthylamine pour obtenir le dérivé de cyclopeptide de formule (IX) :

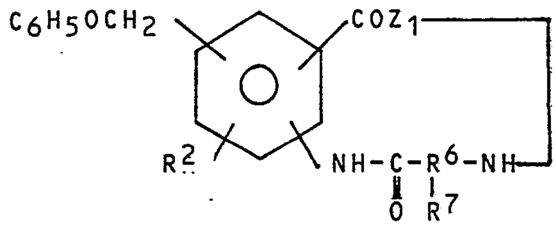

(IX)

A partir du composé de formule (IX), on peut obtenir le composé de formule (I) par réaction avec des réactifs appropriés choisis en fonction de la nature du groupe $R^1$ utilisé.

Dans le cas où $R^1$ est Br, on fait réagir le composé de formule (IX) avec de l'acide bromhydrique et de l'acide acétique ce qui permet d'obtenir le composé de formule (X) :

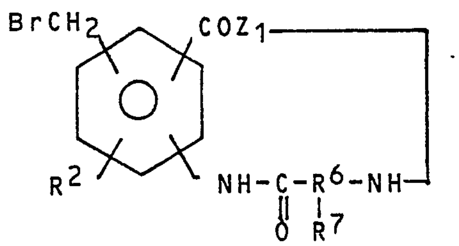

(X)

Dans le cas où l'on veut obtenir un dérivé de cyclopeptide de formule (I) avec $R^1$ représentant le chlore, on fait réagir le dérivé bromé de formule (X) avec de l'acétate de sodium dans du diméthylformamide (DMF), ce qui conduit au dérivé de formule (XI) :

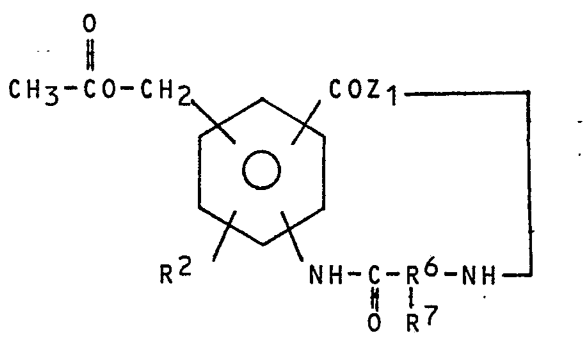

(XI)

que l'on transforme ensuite en dérivé de formule (XII) par réaction avec du méthanol et de la triéthylamine :

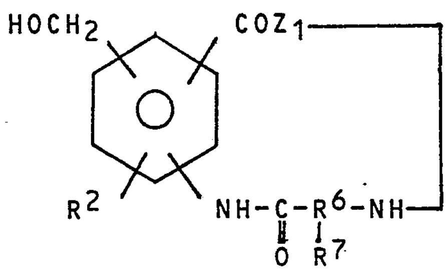

(XII)

En faisant réagir ensuite le dérivé de formule (XII) avec du chlorure de thionyle, on obtient le dérivé chloré de formule (XIII) :

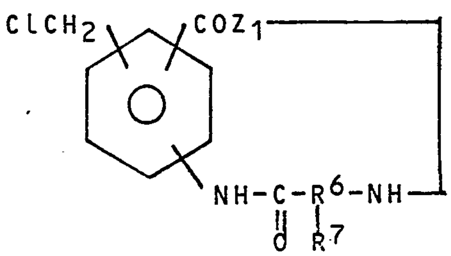

(XIII)

Lorsqu'on veut obtenir le dérivé de cyclopeptide de formule (I) dans laquelle $R^1$ représente $OSO_2R^4$, on fait réagir le dérivé bromé de formule (X) avec le sel d'argent de l'acide sulfonique $R^4$ $SO_3H$, ce qui permet d'obtenir le dérivé de formule (XIV) :

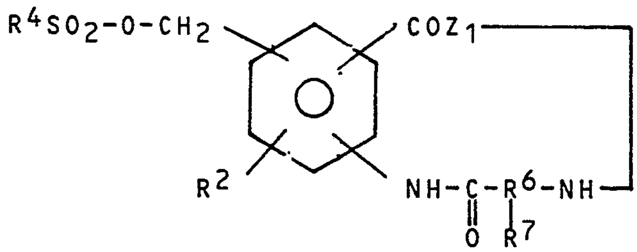

(XIV)

De la même façon un sel d'argent d'un acide phosphinique conduit aux dérivés dans lesquels $R^1 = OP(O)R^4{}_2$ (formule I).

L'action d'un dialkyle sulfure sur le dérivé bromé donne le sel de sulfonium correspondant (I ; $R^1 = S^+R^4{}_2$).

Toutefois, on préfère préparer les dérivés de cyclopeptides dans lesquels $R^1$ représente $S^+R^4{}_2X^-{}_{1/v}$ à partir des dérivés correspondants de formule (IX) par réaction de ceux-ci avec $SR^4{}_2$ et l'acide de formule $XH_v$, selon le schéma réactionnel suivant :

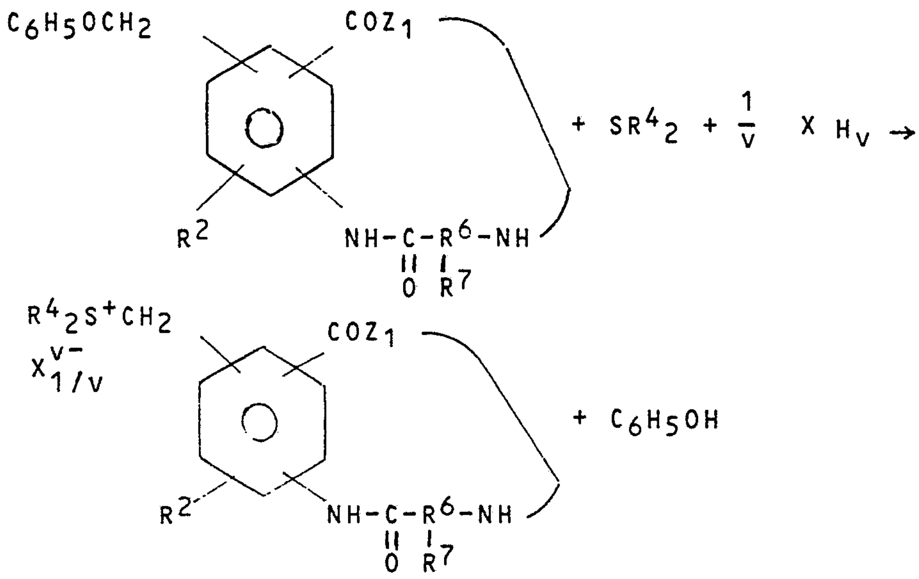


A titre d'exemple $SR^4_2$ peut représenter le thioanisole $CH_3SC_6H_5$ ou le sulfure de diméthyle $CH_3SCH_3$.

Le produit de départ de formule (II)peut être obtenu à partir de l'acide nitrobenzoïque méthylé correspondant, après estérification de la fonction acide, par réaction avec du N-bromosuccinimide pour former le dérivé de formule (XV) :

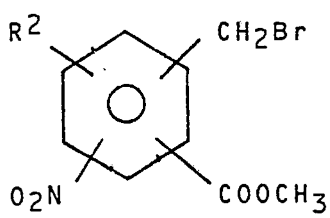

(XV)

que loin fait réagir avec un phénolate de sodium puis avec de la potasse puis avec du $SOCl_2$ en présence de diméthylformamide pour former le composé de formule (II), selon le schéma réactionnel suivant :

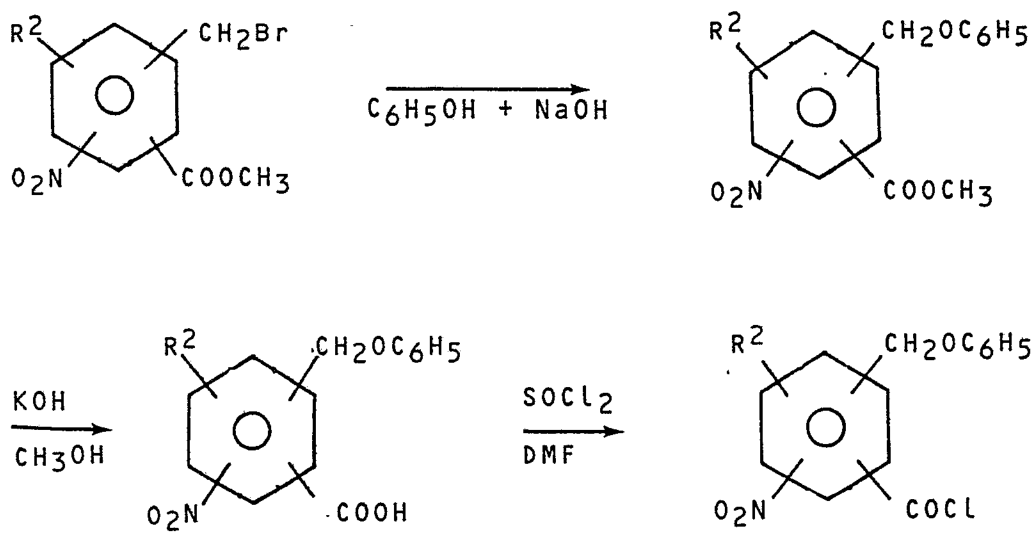


L'invention sera mieux comprise à la lecture des exemples suivants donnés bien entendu à titre illustratif et non limitatif.

<u>Exemple 1</u>

Préparation du dérivé de cyclopeptide de formule :

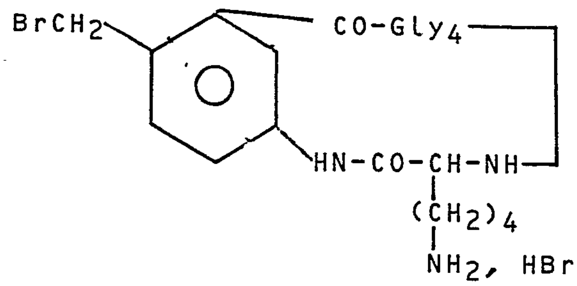

(XVI)

Cette formule correspond à un derivé de cyclopeptide de formule (I) dans laquelle $R^1$ représente le brome, $R^2$ représente un atome d'hydrogène, Z représente $CO(Gly)_4$, $R^4$ représente NH et C(O)-AA est dérivé de la lysine.

1°) Préparation du composé de formule (II) avec $R^2$ = H ; COCl en méta par rapport à $NO_2$ et $C_6NH_5OCH_2$ en para par rapport à $NO_2$.

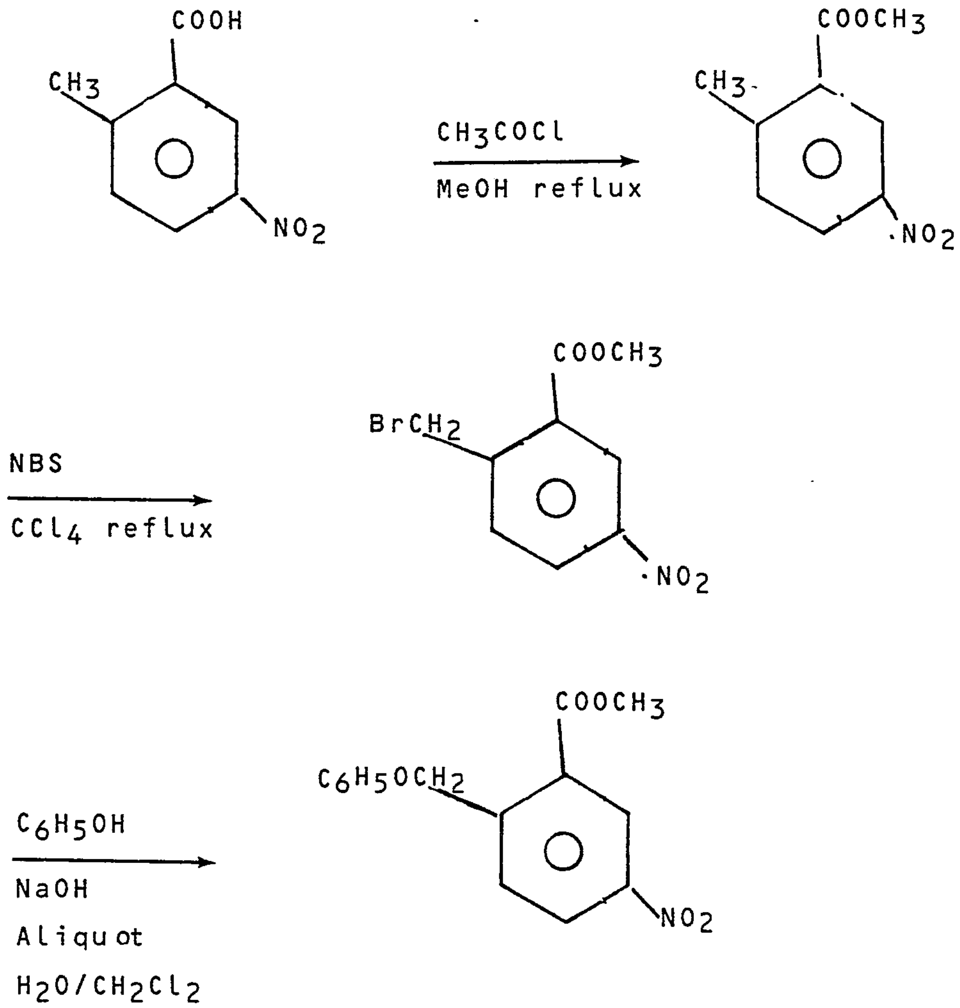

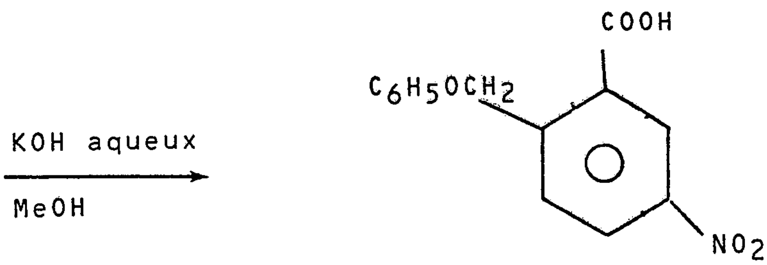


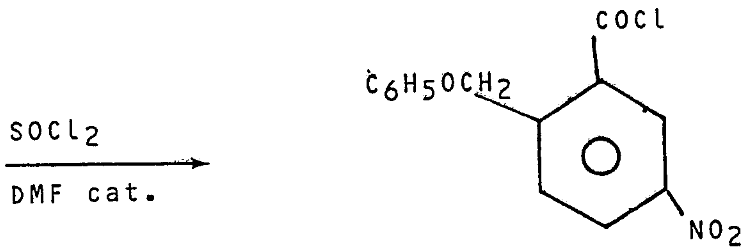


(II)

11,7 g (63,6 mmoles) d'acide nitro-3-méthyl-6-benzoïque sont estérifiés en ester méthylique correspondant par un excès de chlorure d'acétyle dans le méthanol au reflux.

L'ester obtenu est dissous dans 150 ml de $CCl_4$ et traité par 12,5 g (70 mmoles) de N-bromosuccinimide (NBS) et 0,11 g de peroxyde de benzoyle, au reflux pendant 24 heures. Le mélange réactionnel est filtré sur fritté, et le filtrat est évaporé à sec sous pression réduite. Le produit brut obtenu de formule $(BrCH_2)(NO_2)$ $C_6H_3$-$COOCH_3$ est traité directement, sans purification préalable, par 12 g (127 mmoles) de phénol et 2,50 g (6,2 mmoles) d'aliquat (chlorure de tricaprylméthylammonium) dans 250 ml de $CH_2Cl_2$, auxquels on ajoute 5,08 g (127 mmoles) de soude en pastilles dissous dans 250 ml d'eau. Le mélange réactionnel est agité vigoureusement à température ambiante pendant 5 heures. La phase organique est alors séparée, lavée avec 400 ml d'eau, séchée sur $MgSO_4$, filtrée, concentrée à environ 150 ml, et chromatographiée sur une colonne de silice (Kieselgel 60 ; Merck) en éluant avec du chlorure de méthylène ($CH_2Cl_2$). On recueille une fraction d'environ 600 ml qui est évaporée à sec. Le résidu obtenu est dissous dans 150 ml de $CH_2Cl_2$. On ajoute environ 200 ml de méthanol et on porte au reflux. Une cristallisation est observée dans la solution bouillante une fois que tout le $CH_2Cl_2$ est évaporé. Le mélange est encore concentré jusqu'à 150 ml puis laissé à température ambiante pendant une nuit. Les cristaux blancs sont filtrés sur Büchner, abondamment rincés au méthanol et séchés à l'air. On obtient ainsi 11,1 g de cristaux correspondant au dérivé $(C_6H_5OCH_2)(NO_2)$ $C_6H_3$-$COOCH_3$. Le rendement est de 60 % par rapport à l'acide de départ $(CH_3)(NO_2)$ $C_6H_3$-COOH. Point de fusion : 114° C. Spectre $^1$H RMN ($CDCl_3$/TMS) : 8,93, d (J = 2 Hz), 1H ($ArH_1$) ; 8,45, dd (J = 2 Hz et 8 Hz) ; 1H ($ArH_2$) ; 8,07, d (J = 8 Hz), 1H ($ArH_3$) ; 6,8 à 7,5, m, 5H ($C_6H_5O$) ; 5,58, s, 2H ($ArCH_2O$) ; 4,00, s, 3H ($COOCH_3$).

| Analyse élémentaire | C | H | N |
|---|---|---|---|
| Calculé pour $C_{15}H_{13}NO_5$ | 62,71 | 4,56 | 4,88 |
| Trouvé | 62,92 | 4,31 | 4,98 |

On saponifie 3,59 g (12,5 mmoles) d'ester précédent par 25 ml de KOH aqueux 4N (100 mmoles) dans 100 ml de méthanol. Le mélange est agité à température ambiante pendant 4 heures. On ajoute 150 ml d'eau et on évapore le méthanol à 35° C sous pression réduite. La solution aqueuse est extraite avec 100 ml de $CH_2Cl_2$, puis acidifiée par un excès d'acide chlorhydrique concentré. Le précipité résultant est dissous dans 400 ml de $CH_2Cl_2$. La phase organique est séparée, lavée avec 200 ml d'eau, séchée sur $MgSO_4$, filtrée et évaporée à sec, conduisant à 2,79 g d'acide de formule $(C_6H_5OCH_2)$ $(NO_2)$ $C_6H_3$-COOH obtenu sous forme de cristaux jaune pâle. Le rendement est de 82 %. Point de fusion : 170° C. Spectre $^1$H RMN (acétone $d_6$) : 8,93, d (J = 2 Hz), 1H ($ArH_1$) ; 8,55, dd (J = 2 Hz et 8 Hz), 1H ($ArH_2$) ; 8,15, d (J = 8 Hz), 1H ($ArH_3$) ; 6,8 à 7,7, m, 5H ($C_6H_5O$-) ; 5,71, s, 2H ($ArCH_2O$).

| Analyse élémentaire | C | H | N |
|---|---|---|---|
| Calculé pour $C_{14}H_{11}NO_5$ | 61,54 | 4,06 | 5,12 |
| Trouvé | 61,98 | 3,84 | 5,12 |

On traite 2,79 g (10,2 mmoles) d'acide précédent par 50 ml de $SOCl_2$ et 10 gouttes de diméthylformamide (DMF). La solution limpide obtenue est agitée 2 heures à température ambiante puis évaporée à sec sous pression réduite à 40°C. Le résidu obtenu correspond au chlorure d'acide de formule (II) : $(C_6H_5OCH_2)(NO_2)C_6H_3$-CO-Cl et est utilisé directement, sans purification, dans l'étape suivante (2°).

2°) Préparation du composé de formule (XVII) :

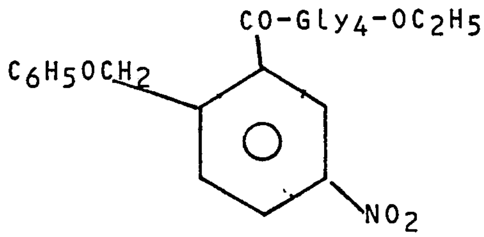

(XVII)

3,57 g (10 mmoles) de composé H-$Gly_4$-$OC_2H_5$, HBr et 50 ml de DMF sont agités et refroidis à 0°C. 3 ml (21 mmoles) de triéthylamine sont ajoutés. Le mélange est agité pendant 10 minutes, puis une solution de 10,2 mmoles de chlorure d'acide (II) avec $R^2$ = H, COCl en méta par rapport à $NO_2$ et $C_6H_5O$-$CH_2$ en para par rapport à $NO_2$, obtenu précédemment (1°), dans 40 ml de DMF, est additionnée goutte à goutte en environ 0,5 heure, à 0°C. Après addition, l'agitation est poursuivie à température ambiante pendant 1 nuit. Le mélange est évaporé à sec à 50°C sous pression réduite. Le résidu obtenu est trituré dans 200 ml d'eau et le précipité résultant filtré sur Büchner, lavé successivement avec 200 ml $H_2O$, 200 ml HCl 0,5 N, 100 ml $H_2O$, 200 ml $NaHCO_3$ 5 %, 200 ml $H_2O$, séché à l'air puis trituré dans 50 ml de méthanol, filtré sur Büchner, rincé au méthanol puis à l'éther et séché à l'air, conduisant à 3,83 g (rendement 72 %) de composé (XVII) dont le point de fusion est de 197-200°C et qui est utilisé tel quel dans l'étape suivante (3°). Un échantillon analytique est préparé à partir de 52 mg d'échantillon précédent, dissous dans 1 ml de DMF, par cristallisation après addition de méthanol aqueux. On obtient 34 mg de cristaux dont le point de fusion est 199-203°C. L'analyse par RMN$^1$H donne les résultats suivants. (DMSO-$d_6$) : 9,09, t (J = 5,6 Hz), 1H (NH Gly) ; 8,42, d (J = 2 Hz), 1H (ArH) ; 8,36, dd (J = 2 Hz et 8,6 Hz), 1H (Ar Hz) ; 8,34, t (masqué), 1H (NH Gly) ; 8,28, t (J 5,8 Hz), 1H (NH Gly) ; 8,23, t (J = 5,8 Hz), 1H (NH Gly) ; 7,88, d (J = 8,6 Hz), 1H ($ArH_3$) ; 7,3, m, 2H ($C_6H_5O$-) ; 7,0, m, 3H ($C_6H_5$-0-) ; 5,39, s, 2H ($C_6H_5O$-$CH_2Ar$) ; 4,06, q (J = 7,1 Hz), 2H (0-$CH_2CH_3$) ; 3,96, d (J = 5,5 Hz), 2H ($CH_2$Gly) ; 3,81, d (J = 5,5 Hz), 4H (2$CH_2$Gly) ; 3,75, d (J = 5,6 Hz), 2H ($CH_2$Gly) ; 1,17, t (J = 7,1 Hz), 3H ($OCH_2CH_3$).

| Analyse élémentaire | C | H | N |
|---|---|---|---|
| Calculé pour $C_{24}H_{27}N_5O_9$, 0,5 $H_2O$ | 53,52 | 5,24 | 13,00 |
| Trouvé | 53,48 | 5,09 | 12,50 |

3°) Préparation du composé de formule (XIX) avec la lysine dont le groupement $\alpha NH_2$ est protégé par un groupement terbutyloxycarbonyle Boc et dont le groupement $\varepsilon NH_2$ est protégé par un groupement benzyloxycarbonyle CBZ.

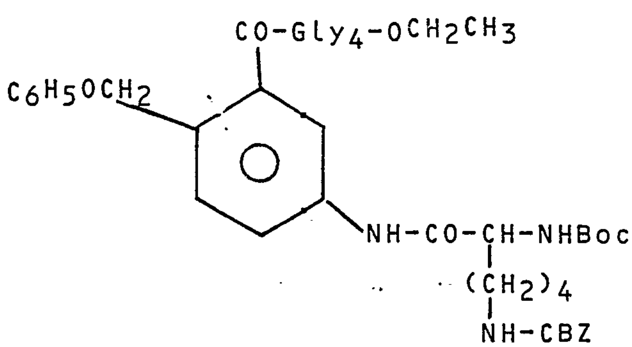

(XIX)

(Suite formule : voir * ci-après).
*(Boc = $(CH_3)_3C$-O-CO-),
(CBZ = $C_6H_5$-$CH_2$-O-CO-).

On dissout tout d'abord 1,06 g (2 mmoles) du peptide de formule (XVII) dans 25 ml de DMF à chaud. On additionne 75 ml de méthanol puis 150 mg (0,7 mmole) de $PtO_2$. On hydrogène le mélange pendant 2 heures à température ambiante, sous environ 3 atmosphères, dans un appareil de Parr. On filtre le catalyseur et on évapore les solvants sous pression réduite. On obtient ainsi le composé de formule (XVIII) :

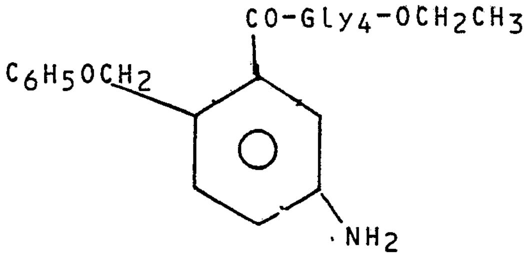

(XVIII)

dont l'analyse chromatographique donne un $R_F$ de 0,46 (support : silice, éluant $CH_2Cl_2$ 85 % - MeOH 15 %). La présence d'une fonction amine aromatique se traduit par une absorption ultraviolette fluorescente (à 366 nm) de la tache chromatographique. Ce composé est peu stable en solution et est donc utilisé immédiatement après sa préparation, sans autre purification, pour la synthèse du composé (XIX).

Ainsi, on dissout l'amine aromatique de formule (XVIII) précédemment obtenue dans 1 ml de DMF, on ajoute 25 ml de $CH_2C_{12}$ et on refroidit la solution à -5°C. On ajoute alors 0,912 g (2,4 mmoles) de N α-Boc-N ε-CBZ-L-lysine, puis 0,495 g (2,4 mmoles) de dicyclohexylcarbodiimide (DCC) dans 25 ml de $CH_2Cl_2$. Le mélange est agité à -5°C pendant 2 heures puis à température ambiante pendant la nuit. On filtre alors le précipité de dicyclohexylurée formé, et on évapore les solvants. Le résidu est dissout dans 400 ml d'acétate d'éthyle et la phase organique est extraite successivement avec 200 ml $NaHCO_3$ 5 %, 200 ml $H_2O$, 200 ml HCl 0,5 N, 200 ml $H_2O$, puis séchée sur $MgSO_4$, filtrée et évaporée à sec. Le peptide obtenu est purifié sur colonne de silice (Kieselgel 60) en utilisant comme éluant un mélange méthanol/$CH_2Cl_2$ dans les proportions 10 %/90 %.

On obtient ainsi le dérivé de formule (XIX) avec un rendement de 71 %. L'analyse chromatographique sur silice donne un $R_F$ de 0,70 (éluant : méthanol 15 % - $CH_2Cl_2$ 85 %) ou de 0,29 (éluant:méthanol 10 % - $CH_2Cl_2$ 90 %).

Le point de fusion est de 72 à 76°C.

Le pouvoir rotatoire est

$$[\alpha]^{25°C}_{546nm} = -9,1°$$

(c 0,5 ; MeOH).

L'analyse par RMN$^1$H donne les résultats suivants : ($CD_3OD$) : 8,07, s, 1H ($ArH_1$) ; 7,81, d (J = 8,3 Hz), 1H ($ArH_2$) ; 7,71, d (J = 8,3 $H_z$), 1H ($ArH_3$) ; 7,51, m, 5H (ArH de CBZ) ; 7,44, m, 2H ($C_6H_5O$-) ; 7,13, m, 3H ($C_6H_5O$-) ; 5,42, s, 2H ($C_6H_5O$-$CH_2$-Ar) ; 5,23, s, 2H ($CH_2$ de CBZ) ; 4,35, m, 1H (CH α Lys) ; 4,29, q (J = 7,1 Hz), 2H ($CH_2$ de OEt) ; 4,24, s, 2H ($CH_2$Gly) ; 4,09, s, 2H ($CH_2$Gly) ; 4,08, s, 2H ($CH_2$Gly) ; 4,07, s, 2H ($CH_2$Gly) ; 3,31, m, 2H ($CH_2$ ε Lys) ; 1,91, m, 2H ($CH_2$ β Lys) ; 1,70, m, 4H ($CH_2$j et $CH_2$ δ de Lys) ; 1,63, s, 9H ($3CH_3$ de Boc) ; 1,40, t (J = 7,1 Hz), 3H ($CH_3$ de OEt).

| Analyse élémentaire | C | H | N |
|---|---|---|---|
| Calculé pour $C_{43}H_{55}N_7O_{12}$ | 59,92 | 6,43 | 11,38 |
| Trouvé | 59,77 | 6,55 | 11,21 |

4°) Préparation du dérivé de formule (XX) :

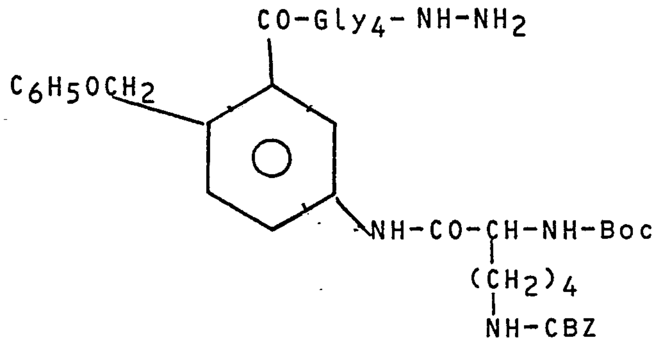

(XX)

On agite 3,2 g (3,7 mmoles) de l'ester de formule (XIX) en solution dans 150 ml de méthanol, en présence de 5,25 ml (108 mmoles) d'hydrate d'hydrazine $H_2NNH_2$, $H_2O$, à température ambiante pendant 12 heures. On évapore la solution sous pression réduite à 40°C. On reprend plusieurs fois le résidu au méthanol et on évapore à sec jusqu'à ce que l'hydrazine ait été totalement entraînée. Le résidu solide est trituré dans l'éther, et le surnageant écarté. Après séchage, on obtient 3,109 g de dérivé (XX) sous forme de poudre blanche. Le rendement est de 98 %. Le point de fusion est de 100 à 110°C.

L'analyse chromatographique sur support de silice donne un RF de 0,46 (éluant méthanol 20 % - $CH_2Cl_2$ 80 %).

Le pouvoir rotatoire est

$$[\alpha]_{546}^{25°C} = -8,4°$$

(c 0,6 ; MeOH).

L'analyse par RMN[1]H donne les résultats suivants : ($CD_3OD$/TMS) : 7,88, d (J = 1,8 Hz), 1H ($ArH_1$) ; 7,62, dd (J = 1,8 Hz et 8,4 Hz), 1H ($ArH_2$) ; 7,53, d (J = 8,4 Hz), 1H ($ArH_3$) ; 7,30, m, 5H (ArH de CBZ) ; 7,24, m, 2H ($C_6H_5O$-) ; 6,94, m, 3H ($C_6H_5O$-)) ; 5,22, s, 2H ($C_6H_5OCH_2Ar$) ; 5,04, s, 2H ($CH_2$ de CBZ) ; 4,15, m, 1H (CH $\alpha$ de Lys) ; 4,06, s, 2H ($CH_2$ de GLy) ; 3,88, s, 2H ($CH_2$ de Gly) ; 3,87, s, 2H ($CH_2$ de Gly) ; 3,84, s, 2H ($CH_2$ de Gly) ; 3,12, m, 2H ($CH_2$ $\varepsilon$ de Lys) ; 1,75, m, 2H ($CH_2$ $\beta$ de Lys) ; 1,51, m, 4H ($CH_2$ $\gamma$ et $CH_2$ $\delta$ de Lys) ; 1,43, s, 9H ($3CH_3$ de Boc).

| Analyse élémentaire | C | H | N |
|---|---|---|---|
| Calculé pour $C_{41}H_{53}N_9O_{11}$, 0,5 $H_2O$ | 57,46 | 6,35 | 14,71 |
| Trouvé | 57,36 | 6,37 | 14,46 |

5°) Préparation du dérivé de formule (XXI) :

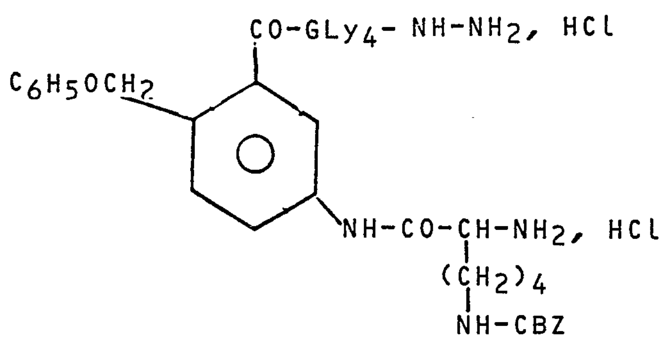

(XXI)

On dissout 3,109 g (3,7 mmoles) du composé de formule (XX) dans 80 ml d'acide acétique glacial, et on ajoute 20 ml d'une solution 2M d'acide chlorhydrique dans l'acide acétique. Aprés 1/2 heure d'agitation, on ajoute 150 ml d'éther éthylique. Le précipité est laissé décanter et le surnageant écarté. Le résidu est rincé trois fois à l'éther éthylique, le surnageant étant à chaque fois écarté après décantation. Le précipité résiduel

est dissous dans 100 ml de méthanol et la solution évaporée à sec sous pression réduite à 40°C. Cette opération est répétée une deuxième fois. Le résidu est trituré dans l'éther éthylique jusqu'à obtention d'une poudre blanche homogène. L'éther surnageant est éliminé et le solide résiduel séché sous vide. On obtient ainsi 2,947 g du dérivé de formule (XXI) sous forme de poudre blanche.

Le rendement est de 98 % et le point de fusion est de 84 - 88°C (décomposition) l'analyse chromatographique sur support de silice donne un RF de 0,07 (éluant :MeOH 20 % - $CH_2Cl_2$ 80 %) ou de 0,62 (éluant : acétate d'éthyle/n-butanol/acide acétique/eau en proportion 1/1/1/1).

Le pouvoir rotatoire est

$$[\alpha]_{546}^{25°C} = +23,0°$$

( c 0,5 ; MeOH).

L'analyse par RMN$^1$H donne les résultats suivants : ($CD_3OD$/TMS) : 7,93, d (J = 2,1 Hz), 1H ($ArH_1$) ; 7,71, dd (J = 2,1 Hz et 8,4 Hz) 1H ($ArH_2$) ; 7,56, d (J = 8,4 Hz), 1H ($ArH_3$) ; 7,31, m, 5H (ArH de CBZ) ; 7,25, m, 2H ($C_6H_5O$-) ; 6,94, m, 3H ($C_6H_5O$-) ; 5,24, s, 2H ($C_6H_5O$-$CH_2$-Ar) ; 5,01, s, 2H ($CH_2$ de CBZ) ; 4,07, s, 2H ($CH_2$ de Gly) ; 4,03, m, 1H (CH α de Lys) ; 3,95, s, 2H ($CH_2$ de Gly) ; 3,91, s, 2H ($CH_2$ de Gly) ; 3,89, s, 2H ($CH_2$ de Gly) ; 3,14, m, 2H ($CH_2$ ε de Lys) ; 1,97, m, 2H ($CH_2$ β de Lys) ; 1,53, m, 4H ($CH_2$ γ et $CH_2$ δ de Lys).

6°) Préparation du cyclopeptide de formule (XXII) :

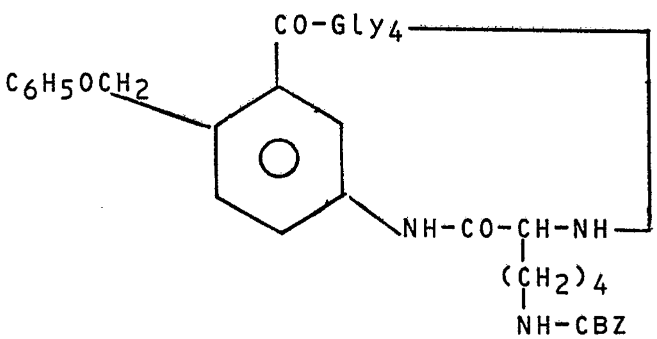

(XXII)

On porte une solution de 0,820 g (1 mmole) du composé de formule (XXI) dans 20 ml de DMF à environ -30 à -40°C. On acidifie ensuite par addition de 1,45 ml (8 mmoles) d'une solution d'acide chlorhydrique 5,5 M dans le THF. On ajoute 0,200 ml (1,5 mmole) de nitrite d'isoamyle. La solution est agitée à -35°C environ pendant 30 minutes, puis diluée par addition de 200 ml de DMF froid. La solution est ensuite alcalinisée jusqu'à un pH de 8-9 par addition de 2,1 $cm^3$ (12 mmoles) de diisopropyléthylamine, agitée à -35°C pendant 10 minutes, puis laissée au repos dans un réfrigérateur (+4°C) pendant 24 heures.

On répète ces opérations plusieurs fois pour traiter au total 3,13 g (3,82 mmoles) de composé de formule (XXI).

On réunit alors les mélanges réactionnels, on ajoute 200 ml d'une solution aqueuse de $K_2CO_3$ à 5 %. On concentre à environ 150 ml, sous pression réduite à 40°C, afin d'éliminer la diisopropyléthylamine, l'eau et une partie du DMF. Le mélange est alors filtré sur fritté pour éliminer les sels minéraux insolubles, et le filtrat est évaporé à sec sous pression réduite à 40°C. Le résidu est une huile qui cristallise. Par addition de 50 ml de méthanol, on obtient des cristaux blancs qui sont filtrés sur Büchner, rincés au méthanol et séchés (poids obtenu : 1,798 g). Le filtrat est évaporé à sec et le résidu chromatographié sur colonne de silice (Kieselgel 60) avec pour éluant une solution de méthanol/$CH_2Cl_2$ dans la proportion 15 %/85 %. Les fractions contenant le produit sont réunies, et les solvants sont évaporés. Le résidu est cristallisé dans du méthanol comme précédemment. On obtient ainsi 0,076 g de cristaux soit au total 1,874 g de cyclopeptide de formule (XXII).

Le rendement est de 68 % et le point de fusion est de 270-280°C (décomposition). L'analyse chromatographique sur support de silice donne un RF de 0,55 (éluant : MeOH 20 % - $CH_2Cl_2$ 80 %) ou de 0,78 (éluant : EtOAc : 1/nBuOH : 1/AcOH : 1/$H_2O$ : 1). La tache correspondante est négative à la ninhydrine.

Le pouvoir rotatoire est

$$[\alpha]_{546}^{25°C} = -40,6°$$

(c 0,5 ; DMF).

L'analyse par RMN$^1$H donne les résultats suivants : (DMSO $d_6$) : 9,03, s, 1H (ArNHCO) ; 8,89, t (J = 5,7 Hz),

1H (NH Gly) ; 8,75, t (J = 4,7 Hz), 1H (NH Gly) ; 8,58, d (J = 7,4 Hz), 1H (NH Lys) ; 8,20, dd (J = 1,6 Hz et 8,4 Hz), 1H ($ArH_2$) ; 8,16, t (J = 6,0 Hz), 1H (NH Gly) ; 7,75, t (J = 5,6 Hz), 1H (NH Gly) ; 7,71, D (J = 2,0 Hz), 1H ($ArH_1$) ; 7,50, d (J = 8,5 Hz), 1H ($ArH_3$) ; 7,34, m, 5H (ArH de CBZ) ; 7,29, m, 2H ($C_6H_5O$-) ; 6,94, m, 3H ($C_6H_5O$) ; 5,21, s, 2H ($C_6H_5OCH_2$-Ar) ; 5,00, s, 2H ($CH_2$ de CBZ) ; 4,20, m, 1H (CH $\alpha$ de Lys) ; 4,10 à 3,60, m, 8H ($4CH_2$ de Gly) ; 3,00, m, 2H ($CH_2$ $\varepsilon$ de Lys) ; 1,93, m, 1H et 1,67, m, 1H ($CH_2$ $\beta$ de Lys) ; 1,41, m, 4H ($CH_2$ $\gamma$ et $CH_2$ $\delta$ de Lys).

| Analyse élémentaire | C | H | N |
|---|---|---|---|
| Calculé pour $C_{36}H_{41}N_7O_9$ | 60,41 | 5,77 | 13,70 |
| Trouvé | 60,40 | 5,70 | 13,43 |

7°) Préparation du cyclopeptide de formule (XVI) :

Un mélange de 0,145 g (0,2 mmole) du cyclopeptide de formule (XXII) et de 20,4 ml d'acide acétique est chauffé jusqu'à obtention d'une solution limpide, puis refroidi à température ambiante. On ajoute 3,6 $cm^3$ d'une solution de HBr à 33 % dans l'acide acétique. Le mélange est agité à température ambiante pendant 1 heure. On ajoute alors 25 $cm^3$ d'éther éthylique, ce qui entraîne la précipitation du cyclopeptide bromé. Après décantation, le surnageant est écarté. Le précipité est agité dans l'éther puis centrifugé, le surnageant étant alors écarté. Cette opération est reproduite de nombreuses fois afin de bien éliminer les acides du milieu. Le précipité est alors séché sous vide. On obtient 0,131 g du cyclopeptide de formule (XVI) sous forme de poudre blanche.

Le rendement est de 99 % et le point de fusion est de 190-200°C (décomposition).

L'analyse chromatographique sur support de silice donne un $R_F$ de 0,43 (éluant : EtOAc : 1/nBuOH : 1/AcOH : 1/$H_2O$ : 1). La tache est négative à la ninhydrine.

Le pouvoir rotatoire est

$$[\alpha]_{546}^{25°C} = 35,5°$$

(c 0,2 ; MeOH).

L'analyse par RMN¹H donne les résultats suivants : ($CD_3OD$) : 8,27, dd (J = 2,2 Hz et 8,4 Hz), 1H ($ArH_2$) ; 7,93, d (J = 2,2 Hz), 1H ($ArH_1$) ; 7,66, d (J 8,4 Hz), 1H ($ArH_3$) ; 5,03, d (J = 10,1 Hz) et 4,98, d (J = 8,1 Hz), 2H ($BrCH_2Ar$) ; 4,59, dd (J = 4,4 Hz et 9,7 Hz), 1H (CH $\alpha$ de Lys) ; 4,38, d (J = 16,9 Hz) et 4,13, d (J = 16,9 Hz), 2H ($CH_2$ de Gly) ; 4,30, s, 2H ($CH_2$ de Gly) ; 4,24, d (J = 16,9 Hz) et 4,13, d (J = 16,9 Hz), 2H ($CH_2$ de Gly) ; 4,15, d (J = 15,8 Hz) et 4,01, d (J = 15,8 Hz), 2H ($CH_2$ de Gly) ; 3,15, t (J = 7,5 Hz), 2H ($CH_2$ $\varepsilon$ de Lys) ; 2,25, m, 1H et 2,03, m, 1H ($CH_2$ $\beta$ de Lys) ; 1,89, m, 2H ($CH_2$ $\delta$ de Lys) ; 1,74, m, 2H ($CH_2$ $\gamma$ de Lys).

| Analyse élémentaire | C | H | N |
|---|---|---|---|
| Calculé pour $C_{22}H_{31}N_7O_6Br_2$ | 40,69 | 4,81 | 15,10 |
| Trouvé | 40,88 | 5,01 | 13,64 |

Spectre de masse (FAB > 0) :568 ($MH^+$ pour $^{79}Br$) ;
570 ($MH^+$ pour $^{81}Br$).

## Exemple 2

On vérifie les propriétés du dérivé de cyclopeptide de formule (XVI), en particulier sa capacité d'inactiver la trypsine bovine ou l'urokinase humaine en utilisant les méthodes de Kitz et Wilson (pour trypsine et urokinase) et la méthode de Hart et O'Brien (pour trypsine) (1973) publiée dans Biochemistry, 12, 2940-2945.

La constante apparente d'inactivation $k_i/K_i$ est dans ce cas de :

- 53 $M^{-1}s^{-1}$ pour la trypsine bovine, et
- 4,2 $M^{-1}s^{-1}$ pour l'urokinase humaine.

En revanche, ce dérivé de cyclopeptide n'a pas d'action sur l'élastase.

## Exemple 3

Préparation du dérivé de cyclopeptide de formule (XXIII) :

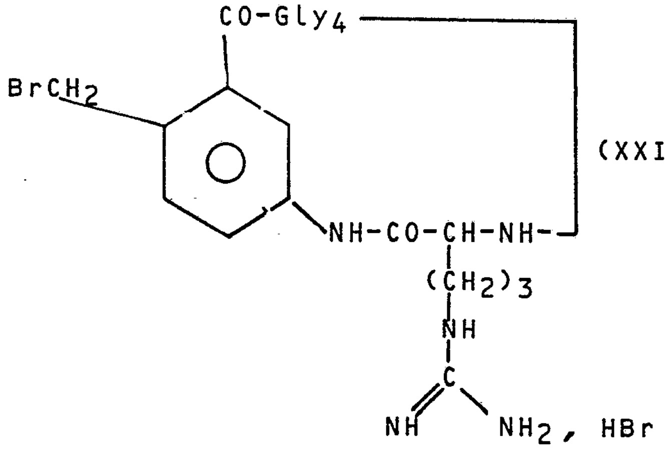

(XXIII)

Cette formule correspond à un dérivé de cyclopeptide de formule (I) dans laquelle $R^1$ représente le brome, $R^2$ représente un atome d'hydrogène, Z représente $CO(Gly)_4$, $R^3$ représente NH et CO -AA est dérivé de l'arginine.

On suit un mode opératoire similaire à celui de l'exemple 1 pour préparer ce dérivé de cyclopeptide sauf que dans la troisième étape, on fait réagir le composé de formule (XVIII) avec l'arginine dont le groupement $\alpha NH_2$ est protégé par un groupement butyloxycarbonyle Boc.

On obtient ainsi le cyclopeptide de formule (XXIII). Celui-ci présente les caractéristiques suivantes :

$R_F$ = 0,52 (support : silice, éluant : EtOAc : 1/nBuOH : 1/AcOH : 1/$H_2O$ : 1).

Point de fusion : 205-215°C (décomposition).

Pouvoir rotatoire :

$$[\alpha]_{546}^{25°C} = -210°$$

(c 0,2 ; MeOH).

Analyse par rénonance magnétique nucléaire : (CD3OD) : 8,25, dd (J = 2,0 Hz et 8,4 Hz), 1H ($ArH_2$) ; 7,96, d (J = 2,0 Hz), 1 H ($ArH_1$) ; 7,66, d (J = 8,4 Hz), 1H ($ArH_3$) ; 5,01, s, 2H ($BrCH_2Ar$) ; 4,61, dd (J = 4,7 Hz et 9,2 Hz), 1H (CH $\alpha$ de Arg) ; 3,9 à 4,5, m, 8H (4$CH_2$ de Gly) ; 3,45, m, 2H ($CH_2$ $\delta$ de Arg) ; 2,28, m, 1H et 2,06, m, 1H ($CH_2$ $\beta$ de Arg) ; 1,93, m, 2H ($CH_2$ $\gamma$ de Arg).

Spectre de Masse (FAB > 0) : 596 ($MH^+$ pour $^{79}Br$) et 598 ($MH^+$ pour $^{81}Br$).

## Exemple 4

On vérifie les propriétés du dérivé de cyclopeptide de formule (XXIII), en particulier sa capacité d'inactiver la trypsine bovine ou l'urokinase humaine en utilisant les mêmes méthodes que dans l'exemple 2 et en opérant à 25°C avec le tampon phosphate de sodium 0,025M, NaCl 0,1M, Tween 80 0,05% (v/v), pH 7,5, pour l'urokinase humaine.

La constante apparente d'inactivation ki/Ki est de 150 $M^{-1}s^{-1}$ pour la trypsine bovine.

La constante apparente d'inactivation ki/Ki est de 165 $M^{-1}s^{-1}$ pour l'urokinase humaine. On vérifie également les propriétés inactivatrices de ce dérivé vis-à-vis du t-PA humain, à 25°C dans le tampon Tris 0,05M, NaCl 0,038M, Tween 80 0,01% (v/v), pH 8,3.

Dans ce cas ki/Ki = 0,3 $M^{-1}s^{-1}$.

En revanche, ce dérivé de cyclopeptide n'a pas d'action sur l'élastase.

## Exemple 5

Préparation du dérivé de cyclopeptide de formule (XXIV) :

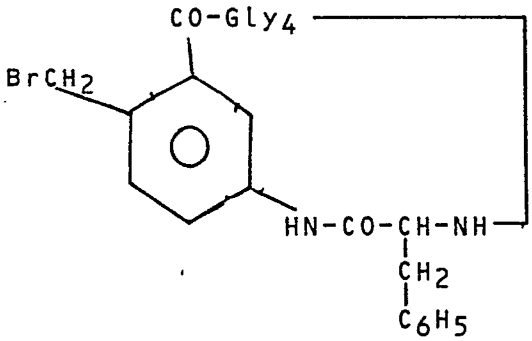

(XXIV)

Ce dérivé de cyclopeptide correspond à un dérivé de formule (I) dans laquelle $R^1$ représente le brome, $R^2$ représente un atome d'hydrogène, Z représente $CO(Gly)_4$, $R^3$ représente NH et CO-AA est dérivé de la phénylalanine.

Pour préparer ce dérivé de cyclopeptide, on suit un mode opératoire similaire à celui de l'exemple 1, sauf que l'on utilise dans la troisième étape la phénylalanine dont le groupement $NH_2$ est protégé par un groupement butyloxycarbonyle Boc.

On obtient ainsi le dérivé de cyclopeptide de formule (XXIV).

Ses caractéristiques sont les suivantes :

$R_F$ = 0,50 (éluant : MeOH 20 % - $CH_2Cl_2$ 80 %) ; 0,69 (éluant : EtOAc : 1/nBuOH : 1/AcOH : 1/$H_2O$ : 1).

Analyse par résonance magnétique nucléaire :

- ($CD_3OD$) 8,15, dd (J = 2,1 Hz et 8,5 Hz), 1H ($ArH_2$) ; 7,92, d (J = 2,2 H), 1H ($ArH_1$) ; 7,66, d (J = 8,5 Hz), 1H ($ArH_3$) ; 7,47, m, 5H (ArH de Phe) ; 5,00, s, 2H ($BrCH_2Ar$) ; 4,86, m, 1H (CH α Phe) ; 3,7 à 42, m, 8H ($4CH_2$ Gly) ; 3,59, m, 1H et 3,20, m, 1H ($CH_2$ de Phe) ;

($CD_3COOD$) 8,03, d (J 8,3 Hz), 1H ($ArH_2$) ; 7,79, s, 1H ($ArH_1$) ; 7,56, d (J = 8,5 Hz), 1H ($ArH_3$) ; 7,36, m, 5H (ArH de Phe) ; 4,93, dd (J = 5 Hz et 10 Hz), 1H (CH α de Phe), 4,86, s, 2H ($BrCH_2Ar$) ; 4,43, d (J = 16,4 Hz), 1H et 4,35, d (J = 16,4 Hz), 1H ($CH_2$ de Gly) ; 4,34, d (J = 16,5 Hz), 1H et 4,17, d (J 16,4 Hz), 1H ($CH_2$ de Gly) ; 4,27, d (J = 16,6 Hz), 1H et 4,10, d (J = 16,5 Hz), 1H ($CH_2$ de Gly) ; 4,09, d (J = 16,0 Hz), 1H et 3,92, d (J = 16, 1 Hz), 1H ($CH_2$ de Gly) ; 3,47, dd (J = 5 Hz et 15 Hz), 1H, et 3,17, dd (J = 10 Hz et 15 Hz), 1H ($CH_2$ de Phe).

Point de fusion : 180-200°C (décomposition).

Pouvoir rotatoire :

$$[\alpha]_{546}^{25°C} = -46,7°$$

(c 0,12 ; DMF).

| Analyse élémentaire | C | H | N |
|---|---|---|---|
| Calculé pour $C_{25}H_{27}N_6O_6Br$, 3,5 $H_2O$ | 46,16 | 5,26 | 12,92 |
| Trouvé | 46,23 | 4,71 | 11,78 |

Spectre de masse (FAB < 0) :586 (Mu pour $^{79}Br$) et 588 (Mu pour $^{81}Br$).

## Exemple 6

On vérifie les propriétés du dérivé de cyclopeptide de formule (XXIV), en particulier sa capacité d'inactiver la chymotrypsine bovine en utilisant les mêmes méthodes que dans l'exemple 2.

La constante apparente d'inactivation $k_i/K_i$ est dans ce cas de 7 $M^{-1}s^{-1}$.

## Exemple 7

Préparation du dérivé de cyclopeptide de formule (XXV) :

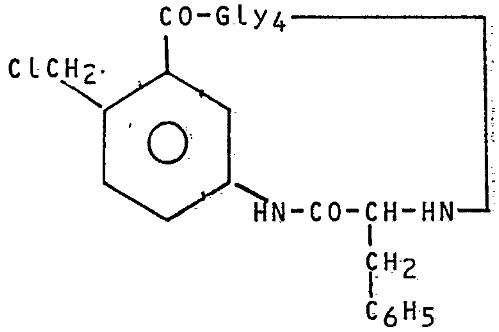

(XXV)

Ce dérivé correspond à un dérivé de cyclopeptide de formule (I) dans laquelle $R^1$ représente Cl, $R^2$ représente un atome d'hydrogène, Z représente CO-$Gly_4$, $R^3$ représente NH et CO-AA est dérivé de la phénylalanine.

On part du dérivé de cyclopeptide de formule (XXIV) et on le traite successivement par de l'acétate de sodium dans du diméthylformamide (DMF) puis par la triéthylamine dans du méthanol et enfin par le chlorure de thionyle.

Dans ce but, 0,025 g (0,04 mmole) de cyclopeptide de formule (XXIV) sont agités vigoureusement à température ambiante, en présence de 0,300 g (3 mmoles) d'acétate de potassium et 3 ml de DMF. Le mélange est ensuite évaporé à sec sous pression réduite. On additionne 25 ml d'un mélange méthanol/chlorure de méthylène 20 : 80, on agite quelques minutes, on filtre et on évapore les solvants. Le résidu est chromatographié sur colonne de silice (Kieselgel 60), un éluant avec un mélange de méthanol (20 %) et de $CH_2Cl_2$ (80 %). Les fractions adéquates sont réunies et les solvants évaporés. Le résidu est dissous dans 2 ml de méthanol. L'addition d'éther provoque une précipitation. Le précipité est centrifugé, rincé à l'éther, centrifugé de nouveau, et séché. On obtient ainsi 0,018 g d'acétate correspondant à la formule (XXVI) :

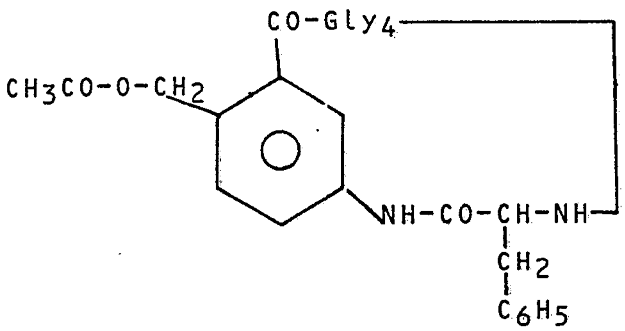

(XXVI)

Le composé de formule (XXVI) ainsi obtenu avec un rendement de 75 % possède les caractéristiques suivantes :

Point de fusion : 190-210°C (décomposition).

$R_F$ (silice) = 0,5 (éluant : MeOH 20 % -$CH_2Cl_2$ 80 %) ; 0,55 (éluant : EtOAc : 1/nBuOH : 1/AcOH : 1/$H_2O$ : 1).

Analyse par résonance magnétique nucléaire : ($CD_3OD$) 8,20, dd (J = 2,1 Hz et 8,4 Hz), 1H ($ArH_2$) ; 7,93, d (J = 2,2 Hz), 1H ($ArH_1$) ; 7,64, d (J = 8,4 Hz), 1H ($ArH_3$) ; 7,47, m, 5H (ArH de Phe) ; 5,49, d (J = 12,9 Hz) et 5,43, d (J = 12,9 Hz), 2H ($AcOCH_2Ar$) ; 4,85, dd (J = 4,6 Hz et 10,1 Hz), 1H (CH α de Phe) ; 4,32, d (J = 16,7 Hz), 1H et 4,09, d (J = 16,9 Hz), 1H ($CH_2$ de Gly) ; 4,25, s, 2H ($CH_2$ de Gly) ; 4,24, d (J = 17,1 Hz), 1H et 4,09, d (J = 16,9 Hz), 1H ($CH_2$ de Gly) ; 4,01, d (J = 15,6 Hz), 1H et 3,82, d (J = 15,7 Hz), 1H ($CH_2$ de Gly) ; 3,60, dd (J = 4,5 Hz) et 14,0 Hz), 1H et 3,18, dd (J = 10,3 Hz et 14,0 Hz), 1H ($CH_2$ de Phe) ; 2,25, s, 3H ($CH_3COO$-).

Pouvoir rotatoire :

$$[\alpha]_{546}^{25°C} = -36,9°$$

(c 0,13 ; DMF).

Spectre de masse (FAB < 0) : 566 (Mu) ; 600 ($M-H-CH_3COOH$)$^-$.

On dissout 0,066 g (0,12 mmoles) de dérivé de formule (XXVI) dans 18 ml de méthanol, on ajoute 2 ml de triéthylamine et on agite la solution à température ambiante pendant 48 heures. Le mélange est alors évaporé à sec à 40°C sous pression réduite, et le résidu chromatographié sur une colonne de silice (Kieselgel 60) en

éluant avec un mélange MeOH : 20 %/$CH_2Cl_2$ : 80 %. Les fractions adéquates sont réunies et la solution concentrée à environ 2 ml. L'addition de 15 ml d'éther éthylique provoque une précipitation. Le précipité est centrifugé, trituré dans l'éther et centrifugé de nouveau, puis séché sous vide. On obtient ainsi 0,043 g de dérivé de formule (XXVII). Le rendement est de 70 %.

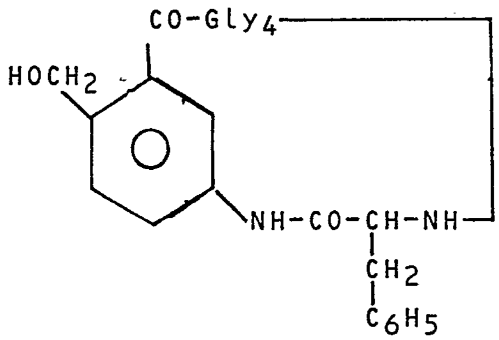

(XXVII)

Le dérivé de formule (XXVII) possède les caractéristiques suivantes :
Point de fusion : 200-220° C (décomposition).
$R_F$ (silice) : 0,50 (éluant : MeOH 20 % - $CH_2Cl_2$ 80 %).
Pouvoir rotatoire :

$$[\alpha]_{546}^{25°C} = - 80,0°$$

(c 0,2 ; DMF).

Analyse par résonance magnétique nucléaire : ($CD_3OD$) : 8,23, dd (J = 2,2 Hz et 8,3 Hz), 1H ($ArH_2$) ; 8,00, d (J = 2,2 Hz), 1H ($ArH_1$) ; 7,63, d (J = 8,3 $H_x$), 1H ($ArH_3$) ; 7,49, m, 5H (ArH de Phe) ; 4,87, s, 2H ($OCH_2Ar$) ; 4,85, dd partiellement masqué (J = 4,4 Hz et estimé 10,6 Hz), 1H (CH α de Phe) ; 4,38, d (J = 16,8 Hz), 1H et 4,11, d (J = 16,8 Hz), 1H ($CH_2$ de Gly) ; 4,30, d (J = 16,3 Hz), 1H et 4,23, d (J = 16, 3 Hz), 1H ($CH_2$ de Gly) ; 4,28, d (J = 17,1 Hz), 1H et 4,00, d (J = 17,1 Hz), 1H ($CH_2$ de Gly) ; 4,03, d (J = 15,4 Hz), 1H et 3,78, d (J = 15,4 Hz), 1H ($CH_2$Gly) ; 3,65, dd (J = 4,3 Hz et 14,2 Hz), 1H et 3,17, dd (J = 10,6 Hz et 14,2 Hz), 1H ($CH_2$ de Phe).

Finalement, on dissout 0,0157 g (0,03 mmole) du dérivé de formule (XXVII) dans 0,5 ml de DMF. La solution est agitée à 0° C, et on y additionne 0,25 ml d'une solution fraîchement préparée et maintenue à 0° C, de 0,17 ml de $SOCl_2$ dans 5 ml de DMF. Le mélange est agité 1 minute à 0° C puis 3 minutes à température ambiante, et aussitôt évaporé à sec sous pression réduite.

On répète ces opérations plusieurs fois pour traiter au total 0,0443 g (0,08 mmole) de dérivé de formule (XXVII).

Les résidus obtenus après évaporation des solvants sont réunis.

On purifie par chromatographie sur colonne de silice, en éluant avec un mélange MeOH 15 % -$CH_2Cl_2$ 85 %. Les fractions adéquates sont réunies et la solution évaporée à sec. Le résidu est dissous dans 2 ml de méthanol et précipité par addition d'éther éthylique. Le précipité est centrifugé, trituré dans l'éther, centrifugé de nouveau et séché sous vide. On obtient ainsi 0,0268 g de chlorure de formule (XXV). Le rendement est de 58 %. Le dérivé de formule (XXV) possède les caractéristique suivantes :
Point de fusion : 170-230° C (décomposition à l'état solide).
$R_F$ (silice) : 0,67 (éluant : MeOH 20 %-$CH_2Cl_2$ 80 %) ; 0,74 (éluant : EtOAc : 1/nBuOH : 1/AcOH : 1/$H_2O$ : 1).
Pouvoir rotatoire :

$$[\alpha]_{546}^{25°C} = -31,8°$$

(c 0,8 ; MeOH).

Analyse par résonance magnétique nucléaire : ($CD_3OD$) : 8,17, dd (J = 2,1 Hz et 8,4 Hz), 1H ($ArH_2$) ; 7,94, d (J = 2,1 Hz), 1H ($ArH_1$) ; 7,68, d (J = 8,4 Hz), 1H ($ArH_3$) ; 7,48, m, 5H (ArH de Phe) ; 4,86, dd (J = 4,7 $N_z$ et 10,0 Hz), 1H (CH α de Phe) ; 4,83, s, 2H ($ClCH_2Ar$) ; 4,32, d (J = 17,0 Hz), 1H et 4,12, d (J 17,0 Hz), 1H ($CH_2$ de Gly) ; 4,29, s, 2H ($CH_2$ de Gly) ; 4,23, d (J = 16,9 Hz), 1H et 4,09, d (J = 16,8 Hz), 1H ($CH_2$ de Gly) ; 4,01, d (J = 15,8 Hz), 1H et 3,85, d (J 15,8 Hz), 1H ($CH_2$ de Gly) ; 3,59, dd (J = 4,7 Hz et 14,0 Hz), 1H et 3,19, dd (J = 10,1 Hz et 14,0 Hz), 1H ($CH_2$ de Phe).

| Analyse élémentaire | C | H |
|---|---|---|
| Calculé pour $C_{25}H_{27}N_6O_6Cl$, 1,5 $H_2O$ | 52,68 | 5,31 |
| Trouvé | 52,43 | 4,69 |

Spectre de masse (DCI) :542 (M° pour $^{35}Cl$) ; 544 (M° pour $^{37}Cl$).

## Exemple 8

On vérifie les propriétés du dérivé de cyclopeptide de formule (XXV), en particulier sa capacité d'inactiver la chymotrypsine bovine en utilisant les mêmes méthodes que dans l'exemple 2.

La constante apparente d'inactivation $k_i/K_i$ est dans ce cas de 2,8 $M^{-1}s^{-1}$.

Les résultats des exemples 2,4,6 et 8 sont récapitulés dans le tableau 1 annexé.

## Exemple 9

Préparation du cyclopeptide de formule (XXVIII).

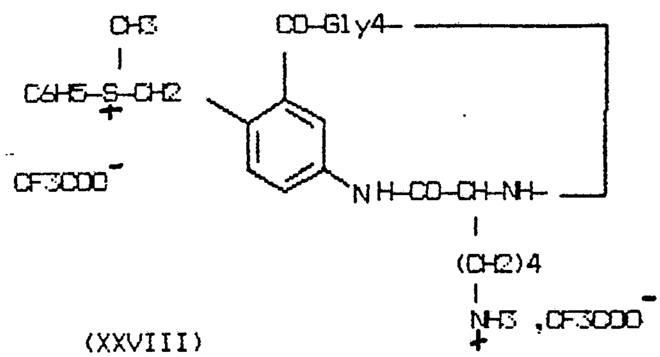

(XXVIII)

Une solution de 0,100g (0,14 mmole ) de cyclopeptide de formule (XXII) et de 1,412 $cm^3$ (14 mmoles) de thioanisole dans 5,6$cm^3$ d'acide trifluoroacétique (TFA), est agitée à température ambiante pendant 24h. On additionne alors 50$cm^3$ d'éther éthylique, ce qui provoque la formation d'un précipité blanc. Le précipité est décanté et le surnageant écarté. Le précipité est rincé plusieurs fois à l'éther, collecté par centrifugation, séché sous vide, et purifié par chromatographie sur colonne de silice (Kieselgel 60) en éluant avec une solution EtOAc/nBuOH/AcOH/$H_2O$ 1:1:1:1. Les fractions adéquates sont réunies et la solution évaporée à sec sous pression réduite. Le résidu obtenu est dissous dans 2$cm^3$ d'acide trifluoroacétique, puis précipité à l'éther. Le précipité est rincé plusieurs fois à l'éther, collecté par centrifugation et séché. Il est dissous dans 2$cm^3$ de méthanol, reprécipité. à l'éther, rincé plusieurs fois à l'éther, collecté par centrifugation et séché. Il est alors dissous dans 10$cm^3$ d'eau. La solution est filtrée, puis lyophilisée. On obtient ainsi 0,056g de cyclopeptide de formule (XXVIII) sous forme de poudre blanche. Le rendement est de 48% et le point de fusion est de 135°C (avec décomposition). L'analyse chromatographique sur support de silice donne un Rf de 0,12 (éluant EtOAc/nBuOH/AcOH/$H_2O$ 1:1:1:1). Le pouvoir rotatoire est $(\alpha)^{25°C}$(546nm) = -25.8° (cO,2;$H_2O$). L'analyse par RMN$^1$H donne les résultats suivants ($D_2O$) : 7,8-7,3, m, 3H ($ArH^1H^2H^3$) ; 7,60, m, 5H ($C_6H_5S^+$); 5,13, m, 2H ($ArCH_2S^+$) ; 4,26, m, 1H (CH α Lys); 3,94, m, 8H (4 $CH_2$ Gly) ; 3,29, s, 3H ($CH_3S^+$); 2,96, m, 2H ($CH_2$ ε Lys) ; 1,9-1,4, m, 6H ($CH_2\beta\gamma\delta$ Lys).

## Exemple 10

Préparation du cyclopeptide de formule (XXIX)

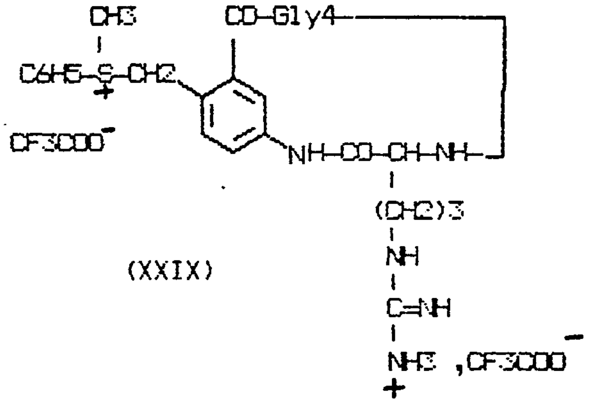

(XXIX)

Cette formule correspond à un dérivé de cyclopeptide de formule (I) dans laquelle $R^1$ représente $C_6H_5S^+(CH_3)$-, $CF_3COO^-$ $R^2$ ; représente un atome d'hydrogène, Z représente $CO(Gly)_4$, $R^3$ représente NH et C(O)-AA est dérivé de l'arginine. On suit un mode opératoire similaire à celui des exemples 1 (étapes 1 à 6) et 9, sauf que dans la 3éme étape de l'exemple 1, on fait réagir le composé de formule (XVIII) avec l'arginine dont le groupement $\alpha NH_2$ est protégé par un groupement butyloxycarbonyle Boc. On obtient ainsi le cyclopeptide de formule (XXIX). Celui-ci présente les caractéristiques suivantes : Rf = 0,14 (support silice ; éluant EtOAc/nBuOH/AcOH/$H_2O$ 1:1:1:1). Point de fusion 125-130°C (avec décomposition). Pouvoir rotatoire $(\alpha)^{25°C}$(546nm) = -4°(c0,1 ; $H_2O$). Analyse par résonance magnétique nucléaire $^1H$ ($D_2O$): 7,8-7,3, m, 3H ($ArH^1H^2H^3$); 7,61, m, 5H ($C_6H_5S^+$); 5,10, m, 2H ($ArCH_2S^+$); 4,28, m, 1H (CH $\alpha$ Arg); 3.95, m, 8H (4 $CH_2$ Gly); 3,30, s, 3H ($CH_3S^+$); 3,20, m, 2H ($CH_2\delta$ Arg); 2,0-1,6, m, 4H ($CH_2$ $\beta\gamma$ Arg).

Exemple 11

Préparation du cyclopeptide de formule (XXIX').

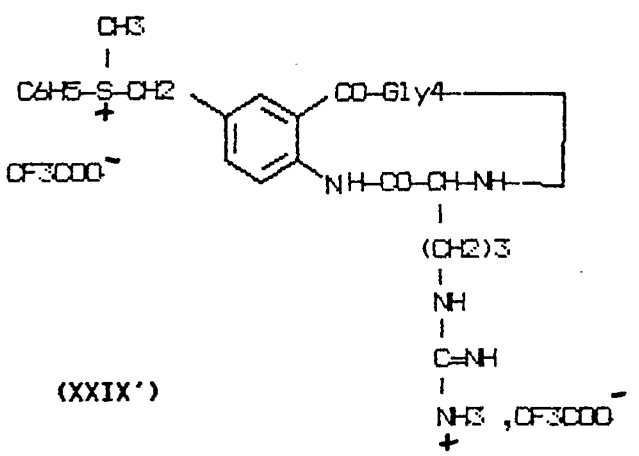

(XXIX')

Ce composé est un isomère de position du composé (XXIX). Sa formule correspond à un dérivé de cyclopeptide de formule (I) dans laquelle $R^1$ représente $C_6H_5S^+(CH_3)$-,$CF_3COO^-$; $R^2$ représente un atome d'hydrogène, Z représente $CO(Gly)_4$ en position ortho (au lieu de meta) par rapport à $R^3$ qui représente NH, et C(O)-AA est dérivé de l'arginine. On suit un mode opératoire similaire à celui de l'exemple 10, excepté que l'arginine dont le groupement $\alpha NH_2$ est protégé par un groupement Boc, est couplée avec le composé de formule (XVIII') obtenu de façon analogue à (XVIII) à partir du composé (II') analogue à (II), lui même obtenu à partir de l'acide nitro-2 méthyl-5 benzoïque.

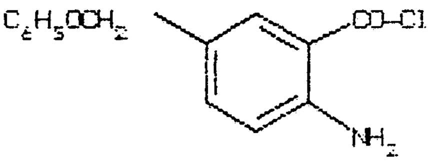

(II')

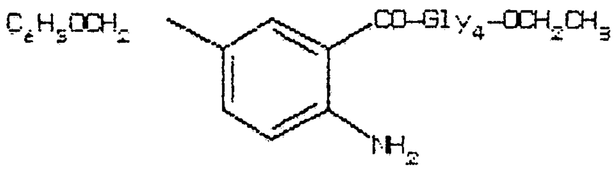

(XVIII')

On obtient ainsi le cyclopeptide de formule (XXIX'). Celui-ci présente les caractéristiques suivantes : Rf = 0,12 (support silice; éluant EtOAc/nBuOH/AcOH/$H_2O$ 1:1:1:1). Point de fusion 40-150°C (avec décomposition). Pouvoir rotatoire $(\alpha)^{25°C}$(546nm) = +7,37° (c0,1; $H_2O$). Analyse par résonance magnétique nucléaire $^1H$ ($D_2O$): 7,7-7,1, m, 8H ($ArH^1H^2H^3$ et $C_6H_5S^+$); 4,60, m, 2H ($ArCH_2S^+$); 4,30, m, 1H (CH α Arg); 3,90, m, 8H(4 $CH_2$ Gly); 3,30, s, et 3,19, s, 3H ($CH_3S^+$); 3,20, m, 2H ($CH_2\delta$ Arg); 2,1-1.5, m, 4H ($CH_2\beta\gamma$ Arg).

## Exemple 12

### Préparation du cyclopeptide de formule (XXX) :

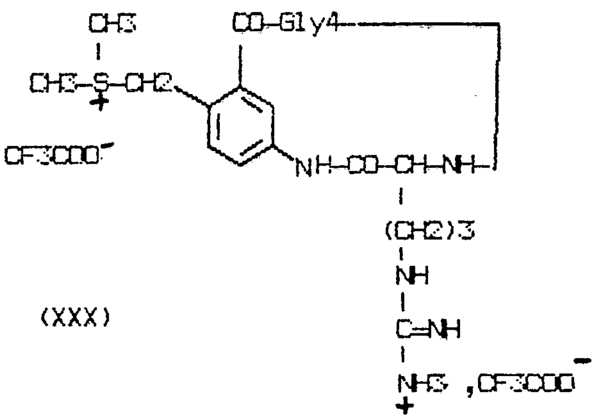

(XXX)

Cette formule correspond à un cyclopeptide de formule (I) dans laquelle $R^1$ représente $(CH_3)_2S^+$-, $CF_3COO^-$; $R^2$ représente un atome d'hydrogène, Z représente $CO(Gly)_4$, $R^3$ représente NH et C(O)-AA est dérivé de l'arginine. On suit un mode opératoire similaire à celui de l'exemple 10, sauf que dans la dernière étape, le thioanisole est remplacé par du sulfure de diméthyle. On obtient ainsi le cyclopeptide de formule (XXX). Celui-ci présente les caractéristiques suivantes : Rf= 0,09 (support silice; éluant EtOAc/nBuOH/AcOH/$H_2O$ 1:1:1:1:1). Point de fusion 130-150°C (avec décomposition). Pouvoir rotatoire $(\alpha)^{25°C}$(546nm) = -25.7° (c0.25; $H_2O$). Analyse par résonance magnétique nucléaire $^1H$ ($D_2O$),. 7,75, dd (J= 2,1Hz et 8.3Hz), 1H ($ArH_2$); 7,63, d (J=2,1Hz), 1H ($ArH^1$); 7,54, d (J=8,3Hz), 1H ($ArH^3$); 4,68, s, 2H ($ArCH_2S^+$) 4,27, m, 1H (CH α Arg); 4,2-3,8, m, 8H (4 $CH_2$ Gly); 3,19, t (J=6,6Hz),2H ($CH_2$ δ Arg); 2,80, s, 6H ($[CH_3]_2S^+$); 2,0-1,5, m, 4H ($CH_2\beta\gamma$ Arg).

## Exemple 13

### Préparation du cyclopeptide de formule (XXXI)

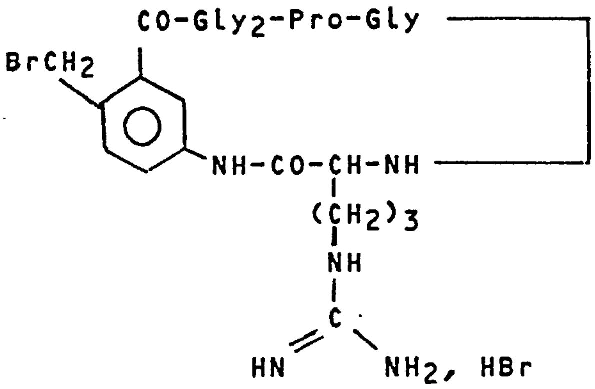

(XXXI)

Cette formule correspond à un dérivé de cyclopeptide de formule (I) dans laquelle $R^1$ représente le brome, $R^2$ représente H, Z représente CO-$Gly_2$-Pro-Gly, $R^3$ représente NH et CO - AA est dérivé de l'arginine.

On suit un mode opératoire identique à celui de l'exemple 3 sauf que dans la deuxième étape on fait réagir le composé de formule (II) avec le composé H-$Gly_2$-Pro-Gly $OC_2H_5$, pour obtenir le composé de formule :

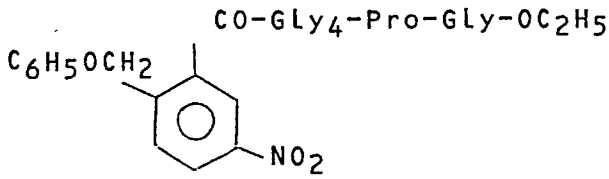

A la fin de la dernière étape, on obtient le cyclopeptide de formule (XXXI).

Le pouvoir rotatoire est

$$[\alpha]^{25^\circ C}_{546\ nm} = -31{,}1\ (c\ 0{,}54\ ;\ H_2O).$$

L'analyse par RMN $^1H$ donne tes résultats suivants : ($D_2O$) : 7,54, d (J = 1,6 Hz), 1H ($ArH_1$) ; 7,51, d (J = 8Hz), 1H ($ArH_3$) ; 7,48, dd (J = 8 Hz et 1,6 Hz), 1H ($ArH_2$) ; 4,65, s, 2H ($CH_2Br$).

## Exemple 14

Les propriétés inactivatrices des cyclopeptides fonctionnalisés de formules XXVIII, XXIX, XXIX′, XXX et XXXI des exemples 9 à 13, répertoriés dans le tableau 2 ont été testées vis-à-vis d'un certain nombre d'enzymes : la trypsine bovine, l'urokinase humaine, le t-PA humain, la plasmine humaine, l'élastase pancréatique porcine. Les mesures cinétiques ont été effectuées à 25° C dans les tampons suivants : Tris 0,1 M, $CaCl_2$ 0,01 M, pH 7,2 (trypsine bovine) ; phosphate de sodium 0,025 M, NaCl 0,1 M, Tween 80 0,05% (v/v), pH 7,5 (urokinase humaine); Tris 0,05 M, NaCl 0,038 M, Tween 80 0,01% (v/v), pH 8,3 (t-PA humain), Tris 0,1 M (élastase pancréatique porcine), phosphate de sodium 0,1 M, glycérol 25% (v/v) (plasmine humaine). En utilisant soit la méthode de Hart et O'Brien -méthode notée a (Biochemistry, 1973, 12, 2940-2945) - et(ou) celle de Kitz et Wilson - méthode notée b (J. Biol. Chem., 1962, 237, 32, 3245-3249) -, on a déterminé la constante $k_i$ de premier ordre caractérisant l'inactivation à concentration infinie en substrat et la constante $K_i$ de dissociation du complexe enzyme-inhibiteur. Leur rapport $k_i/K_i$ est une constante apparente de second ordre caractérisant l'efficacité de l'inactivateur. Les résultats obtenus sont donnés dans le tableau 2 qui suit.

Au vu des résultats des tableaux 1 et 2, on remarque que les dérivés de cyclopeptides des exemples 9 à 13 sont de meilleurs inhibiteurs que les bromures correspondants de formule XVI et XXIII des exemples 1 et 3.

Par ailleurs, les dérivés de cyclopeptides des exemples 9 à 12 sont plus stables en solution aqueuse que les dérivés bromés des exemples 1 et 3.

Ainsi, les dérivés de cyclopeptides de l'invention sont des inhibiteurs sélectifs de certaines protéases, et ils peuvent être utilisés à titre de substance active dans des compositions pharmaceutiques qui peuvent trouver une application thérapeutique dans les pathologies évoquées précédemment.

## TABLEAU 1

### PARAMETRES CINETIQUES CARACTERISANT L'INACTIVATION DE PROTEASES A SERINE PAR DES CYCLOPEPTIDES FONCTIONNALISES (25°C)

| Cyclopeptide | Enzyme | $k_i/K_i$ ($M^{-1}s^{-1}$) |
|---|---|---|
| Composé (XXV) de l'exemple 7 | Chymotrypsine bovine | 2,8 |
| Composé (XXIV) de l'exemple 5 | Chymotrypsine bovine | 7 |
| Composé (XVI) de l'exemple 1 | Trypsine bovine | 53 |
| | Urokinase humaine | 4,2 |
| Composé (XXIII) de l'exemple 3 | Trypsine bovine | 150 |
| | Urokinase humaine | 165 |
| | t-PA humain | 0,3 |

## TABLEAU 2

## PARAMETRES CINETIQUES CARACTERISANT L'INACTIVATION DE PROTEASES A SERINE PAR DES CYCLOPEPTIDES FONCTIONNALISES (25°C)

| Cyclopeptide | Enzyme 1) | $k_i/K_i$ ($M^{-1}s^{-1}$) | Méthode 2) |
|---|---|---|---|
| Dérivé (XXVIII) de l'exemple 9 | Trypsine | 490 | a |
| | Urokinase | 145 | b |
| | Plasmine | 53 | b |
| | Elastase | 0 | |
| Dérivé (XXIX) de l'exemple 10 | Trypsine | 824 | a |
| | Urokinase | 1967 | a et b |
| | t-PA | 1,4 | b |
| | Elastase | 0 | b |
| Dérivé (XXIX') de l'exemple 11 | Trypsine | 16,7 | a |
| | Urokinase | 14 | a |
| | Elastase | 0 | b |
| Dérivé (XXX) de l'exemple 12 | Trypsine | 7 | b |
| | Urokinase | 108 | b |
| | Elastase | 0 | b |
| | t-PA | 0 | b |
| Dérivé (XXXI) de l'exemple 13 | Trypsine | 2016 | a |

1) Enzymes :

Trypsine : trypsine bovine
Urokinase : urokinase humaine
t-PA : activateur tissulaire du plasminogène humain
Plasmine : plasmine humaine
Elastase : élastase pancréatique porcine

2) Méthodes :

a : méthode de Hart et O'Brien
b : méthode de Kitz et Wilson

## Revendications

1. Dérivé de cyclopeptide utilisable comme inhibiteur de protéase, caractérisé en ce qu'il répond à la formule :

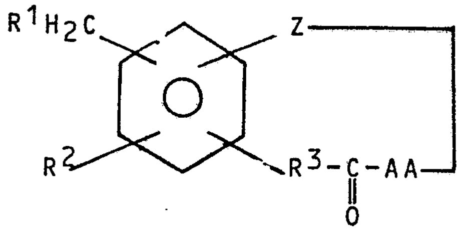

(I)

dans laquelle :

- $R^1$ est choisi parmi les atomes de fluor, de chlore, de brome et d'iode, et les radicaux $OSO_2R^4$, $OP(O)R^4{}_2$, $OC(O)R^4$ et $S^+R^4{}_2\ X^{v-}_{1/v}$, avec $R^4$ représentant un radical alkyle, perfluoroalkyle ou aryle, les $R^4$ pouvant être différents, et $X^-$ représentant un anion de valence v ;
- $R^2$ est un atome d'hydrogène, un radical alkyle, un atome d'halogène, $NO_2$, $COOR^5$, $CF_3$, CN ou $SO_2R^5$ avec $R^5$ représentant un atome d'hydrogène, un radical alkyle ou un radical aryle ;
- $R^3$ représente un atome d'oxygène, un atome de soufre ou -NH- ;
- Z comprend une séquence peptidique $Z_1$ d'aminoacides ou d'analogues identiques ou différents et un groupement choisi parmi $-(CH_2)_n-$, $-O(CH_2)_n-$ ou -CO- à son extrémité liée au noyau aromatique, avec n étant un nombre entier de 1 à 8 ;

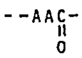

est un radical dérivé d'un aminoacide ou d'un analogue d'aminoacide de formule :

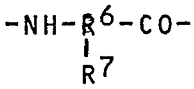

dans laquelle :
$R^6$ représente -N- ou -CH-, et
$R^7$ représente un radical choisi parmi H, $-CH_3$ , $-CH_2CH(CH_3)_2$ ;

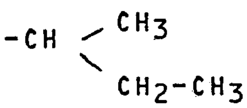

,

$-CH_2-CO-NH_2$, $-CH_2-CH_2-COOH$, $-CH_2-CH_2-CO-NH_2-$, $(CH_2)_4-NH_2$, $-CH_2OH$, $-CH_2-SH$

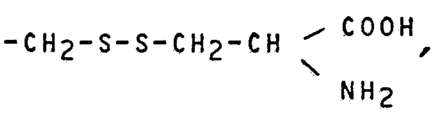

$-CH_2-C_6H_5$,
$-CH_2-C_6H_4OH$, $-(CH_2)_3NHC(=NH)-NH_2$, $-CH_2-COOH$, $-CHOH-CH_3$

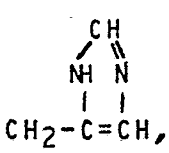

$(CH_2)SCH_3$,

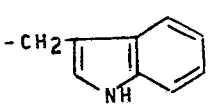

et $-CH(CH_3)_2$
ou de formule :

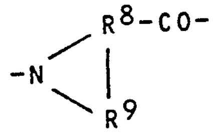

dans laquelle $R^8$ représente N ou CH et $R^9$ représente le radical $-(CH_2)_3-$ ou $-CH_2-CHOH-CH_2$, et ses sels d'addition à un acide pharmaceutiquement acceptable.

2. Dérivé de cyclopeptide selon la revendication 1, caractérisé en ce que la séquence peptidique $Z_1$ comprend 2 à 8 aminoacides ou analogues d'aminoacides identiques ou différents.

3. Dérivé de cyclopeptide selon l'une quelconque des revendications 1 et 2, caractérisé en ce que $R^3$ est en position ortho ou méta par rapport à Z et en position ortho ou para par rapport à $R^1H_2C$.

4. Dérivé de cyclopeptide selon l'une quelconque des revendications 1 à 3, utilisable comme inhibiteur de chymotrypsine, caractérisé en ce que :

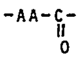

répond à la formule :

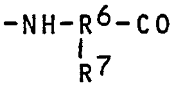

dans laquelle $R^6$ est CH et $R^7$ est $CH_2-C_6H_5$, $CH_2-C_6H_4OH$, $(CH_2)_2SCH_3$ ou :

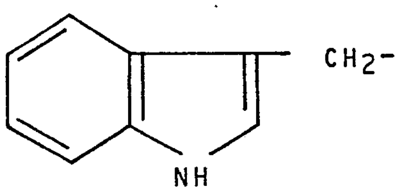

5. Dérivé de cyclopeptide selon l'une quelconque des revendications 1 à 3, utilisable comme inhibiteur de trypsine, caractérisé en ce que -AA-C-(O)- répond à la formule :

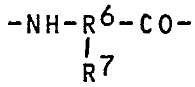

dans laquelle $R^6$ est CH et $R^7$ est $-(CH_2)_4NH_2$ ou $-(CH_2)_3$ NH-C(=NH)-$NH_2$.

6. Dérivé de cyclopeptide selon l'une quelconque des revendications 1 à 3, utilisable comme inhibiteur d'élastase, caractérisé en ce que -AA-C(O)- répond à la formule :

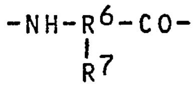

dans laquelle $R^6$ est CH et $R^7$ est $-CH_3$ ou $-CH(CH_3)_2$.

7. Dérivé de cyclopeptide selon l'une quelconque des revendications 4 à 6, caractérisé en ce que $R^1$ est Cl, Br ou $OSO_2R^4$.

8. Dérivé de cyclopeptide selon l'une quelconque des revendications 1 à 6, caractérisé en ce que $R^1$ représente $S^+R^4{}_2X^{v-}_{1/v}$.

9. Dérivé de cyclopeptide selon la revendication 8, caractérisé en ce que $R^1$ représente $C_6H_5\,S^+-CH_3$ ou $CH_3-S^+-CH_3$.

10. Dérivé de cyclopeptide selon l'une quelconque des revendications 4 à 9, caractérisé en ce que $R^2$ est un atome d'hydrogène.

11. Dérivé de cyclopeptide selon l'une quelconque des revendications 2 à 10, caractérisé en ce que Z répond à la formule :

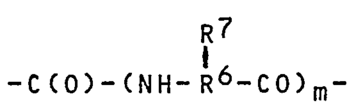

avec m = 4,5 ou 6 et $R^6$ et $R^7$ ayant la signification donnée dans la revendication 1.

12. Composition pharmaceutique ayant la propriété d'inhiber une protéase donnée, caractérisée en ce qu'elle comprend une quantité pharmaceutiquement acceptable d'un dérivé de cyclopeptide selon l'une quelconque des revendications 1 à 11.

13. Composition selon la revendication 12, caractérisée en ce que la protéase appartient au groupe de la trypsine, de la chymotrypsine, de l'élastase ou des protéases à cystéine active.

Office européen des brevets

# RAPPORT DE RECHERCHE EUROPEENNE

Numero de la demande

EP 89 40 2377

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 111, no.11, 11 septembre 1989, page 319, abstract no. 92687r, Columbus, Ohio, US; J.P. MAZALEYRAT et al.: "Directed enzymic hydrolysis of cyclopeptides containing an aminobenzo;c acid residue", & COLLOQ. INSERM 1989, 174(FORUM PEPT., 2nd. 1988), 333 6 * Résumé * --- | 1-13 | C 07 K 5/02<br>C 07 K 7/02<br>C 07 K 5/10<br>C 07 K 7/06<br>A 61 K 37/64 |
| A | CHEMICAL ABSTRACTS, vol. 109, no. 3, 18 juillet 1988, page 662, no. 23365f, Columbus, Ohio, US; J.P. MAYALEYRAT et al.: "Synthesis and enzymic hydrolysis of cyclic peptides containing an anthranilic acid residue", & INT. J. PEPT. PROTEIN RES. 1987, 30(5), 622-33 * Résumé * --- | 1-13 | |
| A | CHEMICAL ABSTRACTS, vol. 109, no. 17, 24 octobre 1988, page 320, no. 145113t, Columbus, Ohio, US; M. REBOUD-RAVAUX et al.: "Structural factors in the enzymic hydrolysis of cyclopeptides containing an o-or m-aminobenzoic acid residue", & BULL. SOC. CHIM. FR. 1988, (2), 267-71 * Résumé * --- | 1-13 | **DOMAINES TECHNIQUES RECHERCHES (Int. Cl.5)**<br>C 07 K<br>A 61 K |
| P,X | CHEMICAL ABSTRACTS, vol. 110, no. 5, 30 janvier 1989, page 259, no. 35964j, Columbus, Ohio, US; A. MOR et al.: "Susceptibility of plasminogen activators to suicide inactivation", & THROMB. RES. 1988, (Suppl. 8), 35-44 * Résumé * ----- | 1-13 | |

Le présent rapport a été établi pour toutes les revendications

| Lieu de la recherche | Date d'achèvement de la recherche | Examinateur |
|---|---|---|
| LA HAYE | 27-11-1989 | MASTURZO P. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)